# EUROPEAN PATENT APPLICATION

(11) **EP 4 582 542 A1**
(43) Date of publication of application: **09.07.2025**
(21) Application number: 23861851.6
(22) Date of filing: 17.03.2023
(51) Int. Cl.: C12N 15/11, C12P 19/34

(54) **METHOD FOR PREPARING OLIGONUCLEOTIDE BY USING RNA LIGASE**

(30) Priority: 08.09.2022 CN 202211107027
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); WANG, Zhaoshuai, Tianjin 300457 (CN); LIU, Fang, Tianjin 300457 (CN); MA, Cuiping, Tianjin 300457 (CN); GENG, Yuhan, Tianjin 300457 (CN); YANG, Yiming, Tianjin 300457 (CN); LI, Juan, Tianjin 300457 (CN); CUI, Lixin, Tianjin 300457 (CN); ZHU, Wenxuan, Tianjin 300457 (CN); JIA, Xu, Tianjin 300457 (CN)
(74) Representative: Delbarba, Andrea
(86) International application number: PCT/CN2023/082298
(87) International publication number: WO 2024/051143

(57) **Abstract**

Provided is a method for preparing an oligonucleotide with a ribonucleic acid (RNA) ligase. The RNA ligase includes any one or more enzymes of RNA ligase families Rnll, Rn12, Rn13, and Rn15, and the oligonucleotide includes natural RNA or non-natural RNA. The method may solve the problem that in the prior art, it is difficult to efficiently synthesize a non-natural RNA strand, and the method is suitable for the field of RNA synthesis.

## Description

The present application is based upon and claims priority to Chinese application No. 202211107027.1, filed on September 8, 2022, the disclosure of which is hereby incorporated again by reference in its entirety.

### Technical Field

The present application relates to the field of ribonucleic acid (RNA) synthesis, and specifically relates to a method for preparing an oligonucleotide with an RNA ligase.

### Background

RNA drugs are a new type of drugs that receive much attention in recent years. Compared to traditional small molecule drugs and new-type protein drugs, the RNA drugs have many advantages. For example, the RNA drugs have strong targeting and only bind to specific sequences on target mRNA. In addition, compared to the development process of the small molecule drugs, the development of the RNA drugs is faster. The RNA drugs may be rapidly modified according to individual differences. The RNA drugs may generally be divided into four categories: RNA aptamer, antisense oligonucleotide (ASO), RNA interference (RNAi), and messenger RNA (mRNA). Herein, RNAi further includes microRNA (miRNA) and small interfering RNA (siRNA). mRNA includes mRNA drugs, mRNA based cell therapies, and mRNA vaccines. In these RNA drugs, the main components of ASO and RNAi are oligonucleotides, which are generally 20-30 bases in length and are currently the most commonly used types of the RNA drugs.

At present, the main method for synthesizing the oligonucleotides in industry is a solid-phase synthesis method. The synthesis of a large number of the oligonucleotides requires many times of accumulations, and the yield is decreased with the increase of the strand length during the synthesis of the oligonucleotides. Meanwhile, as the strand length is increased, impurities in obtained products are also increased, so that the purification process is cumbersome, all of which lead to a significant increase in cost. For RNA with a strand length of tens to hundreds of nt, it is mainly achieved by primer synthesis, and the synthesis scale is limited to the nmol-µmol level, so it is difficult to scale up to the production scale. At present, RNA products with high demands may only be obtained by solid-phase synthesis and many times of cyclic accumulations. For example, to obtain 1 mol of the oligonucleotides, approximately 200 times of solid-phase synthesis accumulations are required. Moreover, due to the characteristics of the solid-phase synthesis, the increase in the strand length may lead to a decrease in purity and yield. When the primer synthesis reaches a strand length of 80 nt, the purity of crude products is only 40%, and the yield is only about 55%.

Many new methods for synthesizing the oligonucleotides are currently under research. Herein, a method for generating an oligonucleotide with a RNA ligase ligation reaction is one of them, this method has the advantages of high efficiency, low cost, and environmental protection and the like. However, RNA enzymes reported in the prior art may not efficiently link to non-natural RNA strands, and non-natural RNA drugs are an important new field in existing pharmaceutical research and development. Developing a fast, efficient, and large-scale production method for the non-natural RNA strands is urgently needed.

### Summary

A main purpose of the present application is to provide a method for preparing an oligonucleotide with an RNA ligase, as to solve the problem in the prior art that it is difficult to efficiently synthesize non-natural RNA strands.

In order to achieve the above purpose, according to a first aspect of the present application, a method for preparing an oligonucleotide with an RNA ligase is provided, and the method includes: an RNA substrate is ligated with an RNA ligase, to obtain an oligonucleotide; and the RNA ligase includes any one or more enzymes from RNA ligase families RnI1, RnI2, RnI3, RnI5, and the oligonucleotide includes natural RNA or non-natural RNA.

Further, the RNA ligase includes any one or more of those having the amino acid sequence as shown in any one of SEQ ID NOs: 1 to 55, or enzymes that have more than 80% sequence identity with any one of SEQ ID NOs: 1~55, preferably 85% or more identity, 90% or more identity, 95% or more identity, 98% or more identity, 99% or more identity, 99.5% or more identity, or 99.9% or more identity; the above method includes: a) a template strand is mixed, annealed, and specifically bound with an RNA substrate, to form a double stranded nucleic acid structure with nicks, and the number of the RNA substrates is 2~10; and b) the nicks are ligated by a phosphodiester bond with the RNA ligase; herein the ribonucleotides ligated by the phosphodiester bond are all non-natural ribonucleotides; preferably, the number of the RNA substrates is 2~3.

Further, each RNA substrate is an RNA fragment of 2~100 nt, preferably 2~10 nt, and more preferably 4~6 nt; preferably, all the ribonucleotides are non-natural ribonucleotides; preferably, the template strand includes a single stranded RNA template or a single stranded DNA template.

In order to achieve the above purpose, according to a second aspect of the present application, a method for preparing a single stranded RNA is provided, and the method for preparing the single stranded RNA includes: a) a single stranded DNA template is mixed, annealed, and specifically bound with an RNA substrate, to form a DNA-RNA hybrid double duplex with nicks, and the number of the RNA substrates is 2~10; b) the nicks are ligated by a phosphodiester bond with an RNA ligase, to form a continuous DNA-RNA hybrid double duplex; c) a DNA strand is removed from the continuous DNA-RNA hybrid double duplex, to obtain a single stranded RNA; herein the RNA ligase includes any one or more enzymes from RNA ligase families RnI1, RnI2, RnI3, RnI5; the ribonucleotides ligated by the phosphodiester bond are all non-natural ribonucleotides. Preferably, the non-natural ribonucleotides include ribonucleotides with one or more of a pentose ring 2'-position modification, a phosphate α-position modification, or a base modification; preferably, the pentose ring 2'-position modification includes but not limited to 2'-methoxyl modification (2'-OCH₃), 2'-fluoro modification (2'-F), 2'-trifluoromethoxyl modification (2'-OCF₃), 2'-methoxyethyl modification (2'-OCH₂CH₂OCH₃), 2'-allyl modification (2'-CH₂CH=CH₂), 2'-amino modification (2'-NH₂) or 2'-azido modification (2'-N₃); preferably, the phosphate α-position modification includes phosphate α-position thio modification (=S); and preferably, the base modification includes methylation modification (-CH₃) and/or acetylation modification (-COCH₃) at any one or more of N1, N5, or N6 positions of the base.

Further, the RNA ligase includes any one or more of those having the amino acid sequence as shown in any one of SEQ ID NOs: 1 to 55, or enzymes that have more than 80% sequence identity with any one of SEQ ID NOs: 1~55, preferably 85% or more identity, 90% or more identity, 95% or more identity, 98% or more identity, 99% or more identity, 99.5% or more identity, or 99.9% or more identity; preferably, a DNA strand in the continuous DNA-RNA hybrid double duplex is removed by DNA enzyme degradation; preferably, the DNA enzyme includes DNasel, DNase1L1, or DNase1L2; preferably, RNA is an RNA fragment with a length of 2~100 nt, preferably 2~10 nt, and more preferably 4~6 nt; preferably, all ribonucleotides are non-natural ribonucleotides; and preferably, the number of the RNA substrates is 2~3.

Further, the single stranded RNA includes a liner single stranded RNA, a semi-circular single stranded RNA or a circular single stranded RNA.

In order to achieve the above purpose, according to a third aspect of the present application, a method for preparing a double stranded RNA is provided, and the method for preparing the double stranded RNA includes: a) a single stranded RNA template is mixed, annealed, and specifically bound with an RNA substrate, to form a double stranded RNA with nicks, and the number of the RNA substrates is 2~10; and b) the nicks are ligated by a phosphodiester bond with an RNA ligase, to form a double stranded RNA; herein the RNA ligase includes any one or more enzymes from RNA ligase families RnI1, RnI2, RnI3, RnI5; the ribonucleotides ligated by the phosphodiester bond are all non-natural ribonucleotides. Preferably, the non-natural ribonucleotides include ribonucleotides with one or more of a pentose ring 2'-position modification, a phosphate α-position modification, or a base modification; preferably, the pentose ring 2'-position modification includes but not limited to 2'-methoxyl modification (2'-OCH₃), 2'-fluoro modification (2'-F), 2'-trifluoromethoxyl modification (2'-OCF₃), 2'-methoxyethyl modification (2'-OCH₂CH₂OCH₃), 2'-allyl modification (2'-CH₂CH=CH₂), 2'-amino modification (2'-NH₂) or 2'-azido modification (2'-N₃); preferably, the phosphate α-position thio modification includes phosphate α-position thio modification (=S); and preferably, the base modification includes methylation modification (-CH₃) and/or acetylation modification (-COCH₃) at any one or more of N1, N5, or N6 positions of the base.

Further, the RNA ligase includes any one or more of those having the amino acid sequence as shown in any one of SEQ ID NOs: 1 to 55, or enzymes that have more than 80% sequence identity with any one of SEQ ID NOs: 1~55, preferably 85% or more identity, 90% or more identity, 95% or more identity, 98% or more identity, 99% or more identity, 99.5% or more identity, or 99.9% or more identity; the single stranded RNA template includes a single stranded RNA prepared with the above method for preparing the single stranded RNA; and the double stranded RNA includes a liner double stranded RNA, a semi-circular double stranded RNA or a circular double stranded RNA.

Further, the RNA substrate is an RNA fragment with a length of 2~100 nt, preferably 2~10 nt, and more preferably 4~6 nt; preferably, the number of the RNA substrates is 2~3.

Further, the ribonucleotides of the RNA substrates are all non-natural ribonucleotides. Preferably, the non-natural ribonucleotides include ribonucleotides with one or more of a pentose ring 2'-position modification, a phosphate α-position modification, or a base modification; preferably, the pentose ring 2'-position modification includes but not limited to 2'-methoxyl modification (2'-OCH₃), 2'-fluoro modification (2'-F), 2'-trifluoromethoxyl modification (2'-OCF₃), 2'-methoxyethyl modification (2'-OCH₂CH₂OCH₃), 2'-allyl modification (2'-CH₂CH=CH₂), 2'-amino modification (2'-NH₂) or 2'-azido modification (2'-N₃); preferably, the phosphate α-position thio modification includes phosphate α-position thio modification (=S); and preferably, the base modification includes methylation modification (-CH₃) and/or acetylation modification (-COCH₃) at any one or more of N1, N5, or N6 positions of the base.

With technical schemes of the present application, any one or more enzymes from the RNA ligase families RnI1, RnI2, RnI3, RnI5 may be used for oligonucleotide preparation. Compared to the prior art of using the solid-phase synthesis method to prepare the oligonucleotide, the efficiency is high and the cost is low.

### Brief Description of the Drawings

Drawings of the description for constituting a part of the present application are used to provide further understanding of the present application. Schematic embodiments of the present application and descriptions thereof are used to explain the present application and do not constitute inappropriate limitations on the present application. In the drawings:
Fig. 1 shows a diagram of a denaturing gel electrophoresis analysis result according to Embodiment 2 of the present application.
Fig. 2 shows an ultra performance liquid chromatography (UPLC) chromatogram according to Embodiment 2 of the present application.
Fig. 3 shows a diagram of a denaturing gel electrophoresis analysis result of an NgrRnl and JN02RnI enzyme reaction according to Embodiment 3 of the present application.
Fig. 4 shows a diagram of an electrophoresis result of an enzyme ligation reaction using NgrRnl according to Embodiment 4 of the present application, herein, in Fig. 4, A shows the effect of substrate concentration on the reaction, B shows the effect of enzyme amount on the reaction, C shows the effect of reaction temperature on the reaction, D shows the effect of adenosine triphosphate (ATP) concentration on the reaction, and E shows the effect of reaction time on the reaction.
Fig. 5 shows a diagram of an electrophoresis result of an enzyme ligation reaction using JN02RnI according to Embodiment 4 of the present application, herein, in Fig. 5, A shows the effect of substrate concentration on the reaction, B shows the effect of enzyme amount on the reaction, C shows the effect of reaction temperature on the reaction, D shows the effect of ATP concentration on the reaction, and E shows the effect of reaction time on the reaction.
Fig. 6 shows a UPLC chromatogram according to Embodiment 4 of the present application.
Fig. 7 shows a UPLC chromatogram according to Embodiment 5 of the present application.
Fig. 8 shows a diagram of an electrophoresis result of catalytic-synthesizing cyclic RNA using RnIARnI according to Embodiment 6 of the present application.

### Detailed Description of the Embodiments

It should be noted that embodiments in the present application and features in the embodiments may be combined with each other without conflicting. The present application is described in detail below in combination with the embodiments.

As mentioned in the background, the synthesis of oligonucleotides in the prior art mainly adopts a solid-phase synthesis method, but there are various disadvantages in solid-phase synthesis. Many new methods for synthesizing the oligonucleotides are currently under research. Herein, a method of generating an oligonucleotide by an RNA ligase ligation reaction is one of them, this method has the advantages of high efficiency, low cost, and environmental protection and the like. However, RNA enzymes reported in the prior art may not be efficiently ligated to non-natural RNA strands, and non-natural RNA drugs are an important new field in existing pharmaceutical research and development. Developing a fast, efficient, and large-scale production method for the non-natural RNA strands is urgently needed. Therefore, in the present application, the inventor attempts to explore an RNA ligation method that uses any one or more enzymes from RNA ligase families RnI1, RnI2, RnI3 and RnI5 to achieve the ligation of non-natural ribonucleotides. Therefore, a series of protection schemes are proposed in the present application.

In a first typical implementation mode of the present application, a method for preparing an oligonucleotide with an RNA ligase is provided, and the method includes: an RNA substrate is ligated with an RNA ligase, to obtain an oligonucleotide; and the RNA ligase includes any one or more enzymes from RNA ligase families RnI1, RnI2, RnI3, RnI5, and the oligonucleotide includes natural RNA or non-natural RNA.

Xeno-nucleic acids (XNA) are a type of nucleic acid molecules that have a non-natural framework or a nucleotide base. The non-natural ribonucleotides refer to ribonucleotides with the non-natural framework or nucleic acid base. The common non-natural ribonucleotides include: (1) a nucleotide with a chemical modification at a 2'-position on a pentose ring, herein the main chemical modification includes 2'-methoxyl, 2'-fluoro, 2'-trifluoromethoxyl and the like; (2) a nucleotide modified on a phosphate, herein it is mainly a thio modification and the like; and (3) a nucleotide that undergoes a chemical modification on a base, herein the main modification includes methylation and acetylation modifications and the like at N1, N5, and N6 positions. RNA containing these types of the non-natural ribonucleotides is called a non-natural RNA. With any one or more RNA ligases shown in SEQ ID NOs: 1~55, the non-natural ribonucleotides are ligated by a phosphodiester bond, to form a strand-like non-natural RNA. With the above RNA ligases, a double stranded RNA with nicks may be ligated, and it may achieve the ligation of very short non-natural RNA, the shortest length of the non-natural RNA may reach 2 nt.

In a preferred embodiment, the RNA ligase includes any one or more of those having the amino acid sequence as shown in any one of SEQ ID NOs: 1 to 55, or enzymes that have more than 80% sequence identity with any one of SEQ ID NOs: 1~55, preferably 85% or more identity, 90% or more identity, 95% or more identity, 98% or more identity, 99% or more identity, 99.5% or more identity, or 99.9% or more identity; preferably, the above method includes: a) a template strand is mixed, annealed, and specifically bound with an RNA substrate, to form a double stranded nucleic acid structure with nicks, and the number of the RNA substrates is 2~10; and b) the nicks are ligated by a phosphodiester bond with the RNA ligase; herein the ribonucleotides ligated by the phosphodiester bond are all non-natural ribonucleotides.

The above RNA ligation method firstly uses the template strand that may specifically bind to the RNA substrate (namely the short RNA single strand) to mix and anneal with 2~10 or even more RNA substrates, and by specific base complementary pairing, binds a plurality of the RNA substrates to the template strand, as to form the double stranded nucleic acid structure with the nicks. The nick between the RNA substrates is discontinuous and lacks the ligation of the phosphodiester bond. With the above RNA ligase, the discontinuous RNA substrates may be ligated by the phosphodiester bond, and the nicks in the double stranded nucleic acid structure are eliminated, thus the continuous double stranded nucleic acid structure is formed. Herein, the above ribonucleotides ligated by the phosphodiester bond are all non-natural ribonucleotides, namely 2 ribonucleotides adjacent to two sides of the nick are both the non-natural ribonucleotides, namely bases at 5'-end and 3'-end of the RNA substrate are non-natural ribonucleotides (the 5'-end of the RNA substrate complementary-paired to the 3'-end of the template strand or the 3'-end of the RNA substrate complementary-paired to the 5'-end of the template strand may not be the non-natural ribonucleotides). However, in the prior art, the RNA ligases with the ability to ligate the non-natural ribonucleotides are rare, and the activity is relatively low.

According to the above principle of the RNA ligase ligating the double stranded RNA with the nicks with a template strand (splint), usually, after 2 short RNA fragments form a complementary strand with the RNA splint, the RNA ligase ligates 5'-phosphate at the nick with 3'-hydroxyl to form the phosphodiester bond. When a plurality of the RNA short fragments complements the RNA splint, the same principle is followed, and the RNA ligase ligates the nicks one by one.

In a preferred embodiment, each RNA substrate is an RNA fragment with a length of 2~100 nt, preferably 2~10 nt, and more preferably 4~6 nt; preferably, all ribonucleotides are non-natural ribonucleotides; preferably, the template strand includes a single stranded RNA template or a single stranded DNA template; and preferably, the number of the RNA substrates is 2~3.

The above RNA substrate may be RNA composed of a minimum of 2 bases and a maximum of 100 bases. The length, quantity, sequence, and other parameters of the RNA substrate may be flexibly adjusted according to factors such as RNA stability, synthesis difficulty, specificity to the template strands, and target non-natural RNA sequences. The RNA substrate includes both the non-natural ribonucleotides and natural ribonucleotides, or may only include the non-natural ribonucleotides. The above template strand used for complementary pairing with the RNA substrate and guiding the arrangement order of the RNA substrates includes a single stranded RNA template or a single stranded DNA template, and the continuous double stranded nucleic acid structure formed is a double stranded RNA or a continuous DNA-RNA hybrid double duplex respectively.

In a second typical implementation mode of the present application, a method for preparing a single stranded RNA is provided, and the method for preparing the single stranded RNA includes: a) a single stranded DNA template is mixed, annealed, and specifically bound with an RNA substrate, to form a DNA-RNA hybrid double duplex with nicks, and the number of the RNA substrates is 2~10; b) the nicks are ligated by a phosphodiester bond with an RNA ligase, to form a continuous DNA-RNA hybrid double duplex; c) a DNA strand is removed from the continuous DNA-RNA hybrid double duplex, to obtain a single stranded RNA; herein the RNA ligase includes any one or more enzymes from RNA ligase families RnI1, RnI2, RnI3, RnI5; the ribonucleotides ligated by the phosphodiester bond are all non-natural ribonucleotides. Preferably, the non-natural ribonucleotides include ribonucleotides with one or more of a pentose ring 2'-position modification, a phosphate α-position modification, or a base modification; preferably, the pentose ring 2'-position modification includes but not limited to 2'-methoxyl modification (2'-OCH₃), 2'-fluoro modification (2'-F), 2'-trifluoromethoxyl modification (2'-OCF₃), 2'-methoxyethyl modification (2'-OCH₂CH₂OCH₃), 2'-allyl modification (2'-CH₂CH=CH₂), 2'-amino modification (2'-NH₂) or 2'-azido modification (2'-N₃); preferably, the phosphate α-position modification includes phosphate α-position thio modification (=S); and preferably, the base modification includes methylation modification (-CH₃) and/or acetylation modification (-COCH₃) at any one or more of N1, N5, or N6 positions of the base.

The above method for preparing the single stranded RNA uses the single stranded DNA template as the template strand and utilizes the above RNA ligase to prepare the continuous DNA-RNA hybrid double duplex. With the DNA enzymes and other methods, one DNA strand in the continuous DNA-RNA hybrid double duplex is degraded, to obtain the single stranded RNA.

In a preferred embodiment, the RNA ligase includes any one or more of those having the amino acid sequence as shown in any one of SEQ ID NOs: 1 to 55, or enzymes that have more than 80% sequence identity with any one of SEQ ID NOs: 1~55, preferably 85% or more identity, 90% or more identity, 95% or more identity, 98% or more identity, 99% or more identity, 99.5% or more identity, or 99.9% or more identity; preferably, a DNA strand in the continuous DNA-RNA hybrid double duplex is removed by DNA enzyme degradation; preferably, the DNA enzyme includes DNasel; preferably, RNA is an RNA fragment with a length of 2~100 nt, preferably 2~10 nt, and more preferably 4~6 nt; preferably, all ribonucleotides are non-natural ribonucleotides; and preferably, the number of the RNA substrates is 2~3.

In a preferred embodiment, the single stranded RNA includes a liner single stranded RNA, a semi-circular single stranded RNA or a circular single stranded RNA.

The semi-circular or circular RNA belongs to the single stranded RNA, its structure is a structure of which a portion complementary to the base is similar to the double stranded, and It does not have a complementary strand, and it is just a free single strand. As long as there are complementary sequences, there is a high probability that they may bind together in solution due to the action of a hydrogen bond, as to form RNA with a partial double stranded structure.

The length of the RNA substrate includes but not limited to 2 nt, 3 nt, 4 nt, 5 nt, 6 nt, 7 nt, 8 nt, 9 nt, 10 nt, 15 nt, 20 nt, 30 nt, 40 nt, 50 nt, 60 nt, 70 nt, 80 nt, 90 nt, or 100 nt.

In a third typical implementation mode of the present application, a method for preparing a double stranded RNA is provided, and the method for preparing the double stranded RNA includes: a) a single stranded RNA template is mixed, annealed, and specifically bound with an RNA substrate, to form a double stranded RNA with nicks, and the number of the RNA substrates is 2~10; and b) the nicks are ligated by a phosphodiester bond with an RNA ligase, to form a double stranded RNA; herein the RNA ligase includes any one or more enzymes from RNA ligase families RnI1, RnI2, RnI3, RnI5; the ribonucleotides ligated by the phosphodiester bond are all non-natural ribonucleotides. Preferably, the non-natural ribonucleotides include ribonucleotides with one or more of a pentose ring 2'-position modification, a phosphate α-position modification, or a base modification; preferably, the pentose ring 2'-position modification includes but not limited to 2'-methoxyl modification (2'-OCH₃), 2'-fluoro modification (2'-F), 2'-trifluoromethoxyl modification (2'-OCF₃), 2'-methoxyethyl modification (2'-OCH₂CH₂OCH₃), 2'-allyl modification (2'-CH₂CH=CH₂), 2'-amino modification (2'-NH₂) or 2'-azido modification (2'-N₃); preferably, the phosphate α-position thio modification includes phosphate α-position thio modification (=S); and preferably, the base modification includes methylation modification (-CH₃) and/or acetylation modification (-COCH₃) at any one or more of N1, N5, or N6 positions of the base.

The above method for preparing the double stranded RNA uses the single stranded RNA template as the template strand and prepares and acquires the double stranded RNA with the above RNA ligase.

In a preferred embodiment, the RNA ligase includes any one or more of those having the amino acid sequence as shown in any one of SEQ ID NOs: 1 to 55, or enzymes that have more than 80% sequence identity with any one of SEQ ID NOs: 1~55, preferably 85% or more identity, 90% or more identity, 95% or more identity, 98% or more identity, 99% or more identity, 99.5% or more identity, or 99.9% or more identity; preferably, the single stranded RNA template includes a single stranded RNA prepared with the above method for preparing the single stranded RNA; preferably, the double stranded RNA includes a liner single stranded RNA, a semi-circular single stranded RNA or a circular single stranded RNA; preferably, the RNA substrate is an RNA fragment with a length of 2~100 nt, preferably 2~10 nt, and more preferably 4~6 nt; preferably, the ribonucleotides of the RNA substrates are all non-natural ribonucleotides; preferably, the number of the RNA substrates is 2~3. Preferably, the non-natural ribonucleotides include ribonucleotides with one or more of a pentose ring 2'-position modification, a phosphate α-position modification, or a base modification; preferably, the pentose ring 2'-position modification includes but not limited to 2'-methoxyl modification (2'-OCH₃), 2'-fluoro modification (2'-F), 2'-trifluoromethoxyl modification (2'-OCF₃), 2'-methoxyethyl modification (2'-OCH₂CH₂OCH₃), 2'-allyl modification (2'-CH₂CH=CH₂), 2'-amino modification (2'-NH₂) or 2'-azido modification (2'-N₃); preferably, the phosphate α-position thio modification includes phosphate α-position thio modification (=S); and preferably, the base modification includes methylation modification (-CH₃) and/or acetylation modification (-COCH₃) at any one or more of N1, N5, or N6 positions of the base.

The above preparation methods for the single stranded RNA and double stranded RNA may be used in combination. For example, the preparation for the single stranded RNA is used firstly, and the single stranded DNA template is used as the template strand, to obtain the single stranded RNA. Then, the single stranded RNA obtained is used as the template strand, to prepare the double stranded RNA. Further, the double stranded RNA is heated so that it is unwound to form 2 single stranded RNAs, which bind to the RNA substrate and repeat the above preparation method to obtain a large amount of the single stranded RNAs or double stranded RNAs. The above preparation method is low in cost and high in efficiency, and has a high application value in industrial production.

The beneficial effects of the present application are further explained in detail below in combination with specific embodiments.

### Embodiment 1: Expression and purification of RNA ligase

Plasmids containing expression sequences of 55 target proteins (the sequences were shown below) were transformed into a competent state of Escherichia coli BL21 (DE3). After expression in the Escherichia coli, the expressed proteins were purified in two steps: an affinity column (Ni-NTA) and an ion column (QFF or SPFF), to obtain 55 proteins with high purity.
SEQ ID NO: 1 (DpRnl, derived from *Diplonema papillatum,* RnI2 family): 
MPLSQGAALIRSPTVTAEEKVDGSNLAVYLDASGAVTCQNRGKFVTPSSGSQWGGQLATWL
ESHYCELVSLLRQRYILYGEWLLARHSIRYQSLPDYFVAFDVYDRTARRFLSAKKRNAFLSQSTIPV
VKPLAVGPFAEADILRLLGSPSRYGAAKVEGIYLRSDSGAWLQQRAKVVNSDFLQTIDDDGHWQK
RVLEKNQLKY.
SEQ ID NO: 2 (AcNPVRnl, derived from *Autographa californica nucleopolyhedrovirus,* RnI2 family): 
MVYICIDTGSHAKGYAVESSDTDYHIYTKCDRETFEKFIDNKELLKNRHAKDESGNDVKYVDL
YTGLIGILTGKSPELSMFSKREDFKDKYGIENLQLYEFVTKLMTVSMVKIIYTLMRYKILNNAKGLLQ
LMFNYVYVEYYLDYKRAPKSTKILNMLFNVGDEIKITMDKNNQLVVNDLNVLDLNKNNGGYKIDETL
TLFVKNVKLLKLYVKLMQRGEYQQEWTEYFQQWKQQLQDRLHHVPEPPERTDIRHNIVMYALNE
RGPVMPEDENKIVYQIYPSVSHLDQGKKGTLADKEIIVQEKLDGCNFRIICNQNKITYGSRNTYRPD
GNFMNYYRIRKDLETCMRSLQARFNDGFIVYGELMGWKDDAKTTPINVINYVDQKESLKYYAYEIQ
LYGGEFVPFVEAQELLTNVGFNTIPCHKYLYNDFVERLNFKSLMFPQSPLEGFIIRCGNLIYKLKSD
YKDLNKLKIEKGPFEWLTCDYIKSNCDAIDKSDMMKILIFCYNMCKVKNYNEKLLFNKVFNLFRQQF
NLNHNDYKNLYKQYVNMCKCTEYK.
SEQ ID NO: 3 (SfRnl, derived from *Shigella flexneri,* RnI2 family): 
MFKKYSSLENHYNSKFIEKLYSLGLTGGEWVAREKIHGTNFSLIIERDKVTCAKRTGPILPAED
FFGYEIILKNYEDSIKAVQDIMETSAVVSYQVFGEFAGPGIQKNVDYGDKDFYVFDIIVTTESGDVTY
VDDYMMESFCNTFKFKMAPLLGRGKFEELIKLPNDLDSVVQDYNFTVDHAGLVDANKCVWNAEV
KGEVFTAEGYVLKPCYPSWMPNGNRVAIKCKNSKFSEKKKSDKPIKAKVELSEADNKLVGILACYV
TLNRVNNVISKIGEIGPKDFGKVMGLTVQDILEETSREGITLTQADNPSLIKKELVKMVQDVLRPAWI
ELVS.
SEQ ID NO: 4 (MthRnl, derived from *Methanothermobacter thermautotrophicus str.* Delta H, Rnl3 family): 
MNSMNSDIPFDLIQERTGVPSSRLKVAFARGSLRLLESAGMQALLFKKPLGDLEAGTVIYLGD
ETEVIRGFPKIRRTLLLSPTIQEHFRDRVAVEEKMNGYNVRIACLSSGETVALTRGGHVCPFTTRKA
QELLDLSEFFREHPDLVICGEMIGRDNPYVSQDYPEVGPLGFRVFDLREKNTNRPLPVEERRALLD
SYGLPNVRLFGVYPIEEAASEVADIIRALGMAGREGVVMKDPSMEVPPLKYTSSQAHARELAYAFS
YPFDFGRPFFFSRVIREGFQAYELDESDDETRERARRLGEAIIYPMLERIKSISAGEAAYEDTVIDVE
DREAAEEFIRHLVRLGVSATLADYRDGRATIRRFYQSTTDRINNYLKGGLY.
SEQ ID NO: 5 (Pab1020Rnl , derived from *Pyrococcus abyssi,* Rnl3 family): 
MVSSVYKEILVKLGLTEDRIETLEMKGGIIEDEFDGIRYVRFKDSAGKLRRGTVVIDEEYVIPGF
PHIKRIINLRSGIRRIFKRGEFYVEEKVDGYNVRVVMYKGKMLGITRGGFICPFTTERIPDFVPQEFF
KDNPNLILVGEMAGPESPYLVEGPPYVKEDIQFFLFDVQEIKTGRSLPVEERLKIAEEYGINHVEVF
GKYTKDDVDELYQLIERLSKEGREGIIMKSPDMKKIVKYVTPYANINDIKIGARVFYELPPGYFTSRIS
RLAFYLAEKRIKGEEFERVAKELGSALLQPFVESIFDVEQEEDIHELFKVRVKRIETAYKMVTHFEKL
GLKIEIVDIEEIKDGWRITFKRLYPDATNEIRELIGGKAFVD.
SEQ ID NO: 6 (NgrRnl, derived from *Naegleria gruberi*, Rnl5 family): 
MSDSEVNQEAKPEVKPEVKPETHINLKVSDGSSEIFFKIKKTTPLRRLMEAFAKRQGKEMDSL
RFLYDGIRIQADQTPEDLDMEDNDIIEAHREQIGGATYMGVRKLATIRTAGEITPIAGAEAIECCHVD
GWTCVIKKGEFKQGDRGVYFEIDSFIKEDNDRYPMLSKQVIDYEGQRGTRLRTARLRGQLSQGLF
LPMDRFPELASNQVGDDVTEILGITKWEPPISTNLSGEILGEFPTFISKTDQERVQNLIPQIEENKGQ
KFEVTVKLDGSSMTVYRKDDHIGVCGRNWELRETATNAQWHAARRNKMIEGLQFLNRNLALQGE
IIGESIQGNLEKLKGQDFYLFDIYDIDKAQYLTPIERQSLVKQLNDNGFTVKHVPILDDLELNHTAEQI
LAMADGPSLNKNVKREGLVFKRLDGKFSFKAISNAYLEKHKDR.
SEQ ID NO: 7 (DraRnl, derived from *Deinococcus radiodurans,* Rnl5 family): 
MAERQVIKERAQLFPHPNAERLELCKVGTFQLVVRKGEYRDGDPIVIAPEKAVLPPQLAGLYT
NADTGASYLHGAEKNRVGSVRLRGEVSQGVILPLDGLEDAPFGEDLAERLGITFWEPPVPVSMAG
EVEPRPPAQHYKHHDVEQFGIYVSEFASGEEVMVTEKLHGTQGVYFRTAEGRWLVTSKGLSRGG
LTLREAASNVYWQAARNSNLFAEADAAFSGGEVQIFGEVVPVQKGFSYGQHKPTVFVFKVVYDG
LRLPRRDWPQWVLDHAVPVLYEGPFDEATVRKLRGGLETVSGKGLHIREGVVVAPKVPRFAADG
SDLSVKLISDAYAKKETGEEYS.
SEQ ID NO: 8 (DdRnl, derived from Dictyostelium discoideum AX4, Rnl5 family): 
MMENIEETLKLVEITDTTTTTTNTTTTNNQEVIIINEKKEIIINENDIEEGRDLDNESGRSLAYFERI
LKLEAIVGADVIEIATVLGWRVVVKKGLYKVGDPVVYCEIDSILPPWQYFIDDKMDSRGFKIKTIKLR
GEISQGYCIPIKELINHPHKKIKAVYKEEEDNNNETIVHLLDEDSNEKIPIEMGRNLTDFIGIKKITEYV
QIPRNVNGSNRKAYTFPSFIKRTDQTRIQSVPHYFDLHQDLLWEVTEKLEGSSITVFKKGKESGVC
SRNFLIEDFQGSDIHQAVIEDLDLLNRLSTIDLNIALQGEILGPKIQGNIYQLRKNYFKIFDIFLIDSQRY
ATHDERIEILNSLKLTPSDHMAPVISNSFSLKGKTLSDILDMANGFSILKESKPKILREGLVFKSTSTI
GSVNQSVVSFKAISNQYLLKKK.
SEQ ID NO: 9 (GzRnl, derived from *Gibberella zeae,* Rnl5 family): 
MPRKLVTVRHISAITPIPGADRIESATVDGWTCVVSTNAFKPGDSGVYFEIDSLLPASDPRFAF
LAPKIVSPNGPSHTPDVRVRTVKIRGVLSQGLLMPLNDFPEIVSRLDTIGSDELQDIGFEDTLNVRK
YDGPATPLSQDSSLGTPLPDFPSFIPRTEQERVQNLPDVFSTHGSEIFQESTKMDGSSMTIENHPR
SQPLFYATARALGLHQTLAKIGRNVAIQGELCGSSVQSNLEGFAIGTHSFFLFAVYDIDDQRYLPPK
EVHEIWAPLLGVKHVPVHGYRALNQVGSAVTDLVARAEGKGVNGRKREGIVFKRDDGLFSFKAIS
NSYLLKHGE.
SEQ ID NO: 10 (NcRnl, derived from *Neurospora crassa,* Rnl5 family): 
WTVVGFTSQMNRFKIGDLVVFMEIDSFIPRIDRYWELWSSMDDVFNGEIGLRVRSREVSGTY
SQGMIFSLADFPEIVLHHQNRIQKIGKDEATRELLSYSFADSLGIKKWEYPTHVRPVTGIIGELSPLIQ
RPGNYRIQDIGNNVFNTHAAKNRIYQVTEKLDGVTMHVYKVSNQSPDLLAYFPALKPTAGSIPIPPT
MQTPRGRVGVCNRKHEFFDDGKNIYWETAKTSGILDKIHKIPYRNIAIQGELVGSHIQGNTMQYPE
GKHEFVVFGIWDMDARDYLATKSVELLCKTLDIAHVPVLGYGPVTKYGKSVEEILAYADKLGPGEY
GGVKEGLIFRANDDWKKGFKVISNGWLKMTGK.
SEQ ID NO: 11 (PoRnl, derived from *Pyricularia oryzae 70-15,* Rnl5 family): 
MEGDSPVVAVTSPTIPTDSTLAVSPTAEKAASTESFSAPAVRKLVTLRRISRIRPVKNAKLPKSK
YVAITVDGWEVVVNAKRDQRRFREGQLVVFFQIDSFVPENDGNFWEEMSTSAELFQERRGFRIGT
LRIGESSLISQGRVFHLGEYPRVEEVVAELQKVHGDKDGLEVAMQRSFDDVLGVVKWDVPEEHK
GAHYGPPPPMIPRTALNRIQDVTQQLWERHAATRVQITEKLDSCPISIYFVRNDSPFAKCLPALHDF
AGKPDAQRRQQQLCVFPHGRVGVCANRLEYVCAPGQRFWAVVERLGIPGRLAREGRTAVVQGE
LVAPDRGGPGGFYAFSLWTEGGKHGGFDGEETQRAALRRMGVPLVPLVGYRAIGEFASSTGEML
AKADGRGFRGQLREGFVLKNVKENIWVKVISNAWLEWYEE.
SEQ ID NO: 12 (SppRnl, derived from *Shigella phage* pSs-1, RnI2 family): 
MFKKYSSLENHYNSKFIEKLYSLGLTGGEWVAREKIHGTNFSLIIERDKVTCAKRTGPILPAED
FFGYEIILKNYADSIKAVQDIMETSAVVSYQVFGEFAGPGIQKNVDYGDKDFYVFDIIVTTESGDVTY
VDDYMMESFCNTFKFKMAPLLGRGKFEELIKLPNDLDSVVQDYNFTVDHAGLVDANKCVWNAEA
KCEVFTAEGYVLKPCYPSWLPNRNRVAIKCKNSKFSEKKKSDKPIKAKVELSEADNKLVGILACYV
TLNRVNNVISKIGEIGPKDFGKVMGLTVQDILEETSREGITLTQADSPSLVKKELVKMVQDVLRPAW
IELVS.
SEQ ID NO: 13 (BpRnl, derived from *Buttiauxella phage vB_ButM_GuL6,* RnI2 family): 
MFVKYSSLTNHYEGKFINGVIMNGLTGGVWVAREKIHGANFSLITSDGEKVIPAKRSGEILPTE
QFYGCEPVVARYAPAIRKLWDVINTAQQVSGIYVDSLVIQVYGEFAGRGVQKDVDYGEKDFYVFDI
 
RVNGEFLHDNIVAVYAKSVGLKMAPLLAYGTFDEIRALPITFDSVVNLANSGAIPAHNGVEPEFKNF
MTLKDGEGENIAEGFVMKPVHPAFMPNGERVAIKCKTTKFSEKKNKQANRFNAPAELSENDKAKL
DVFTCYLTENRVKNVLSKIDSANLTAKDFGRVMGLTVQDALEEIERNYGPFIEQFENPTLAKKTFTN
EASSLIRTNWGAILNNEF.
SEQ ID NO: 14 (TbgRnl, derived from *Trypanosoma brucei gambiense* DAL972, RnI2 family): 
MLRCLGVRHFRRTPLLFVGGDGSIFERYTEIDNSNERRINALKGCGMFEDEWIATEKVHGAN
FGIYSIEGEKMIRYAKRSGIMPPNEHFFGYHILIPELQRYVTSIREMLCEKQKKKLHVVLINGELFGG
KYDHPSVPKTRKTVMVAGKPRTISAVQTDSFPQYSPDLHFYAFDIKYKETEGGDYTTLVYDEAIEL
FQRVPGLLYARAVIRGPMSKVAAFDVERFVTTIPPLVGMGNYPLTGNWAEGLVVKHSRLGMAGF
DPKGPTVLKFKCTAFQEISTDRAQGPRVDEMRNVRRDSINRAGVQLPDLESIVQDPIQLEASKLLL
NHVCENRLKNVLSKIGTEPFEKEEMTPDQLATLLAKDAMKDFLKDTEPSIVNIPVLIRKDLTRYVIFE
SRRLVCSQWKDILKRQSPDFSE.
SEQ ID NO: 15 (F48Rnl, derived from *Klebsiella phage* vB_Kpn_F48, Rnl1 family): 
MFEKYSSLENHYNNRFIEKIRHHGYDVTEGWCAREKIHGTNFSIIIERDAVTCAKRTGPILPAE
DFFGYTVILKKYNDSIKAVQHTIKEGSSMQIFGEFAGSGIQKGVDYGEKDFYVFDILVKTDKGTHQF
VDDFMMEQMCCTFGFKVAPLMGRGSFDELAKLPNEFQINVNRYNEAAEVDLRNANTRVWPAEE
ATDNTAEGYVLKPNYPRFLANGSRVAIKCKNSKFSEKAKSDKPIKPKAVLSEIDQEVLQKFSEYVTI
QRVNNVISKIGQVGPKDFGKVMGLTVQDILVEAGREGLEVIQAEQPDIVKKEITKLVQDVLRPAWIE
LVSN.
SEQ ID NO: 16 (PhRnl, derived from *Pyrococcus horikoshii* OT3, Rnl3 family): 
MVVPLKRIDKIRWEIPKFDKRMRVPGRVYADEVLLEKMKNDRTLEQATNVAMLPGIYKYSIVM
PDGHQGYGFPIGGVAAFDVKEGVISPGGIGYDINCGVRLIRTNLTEKEVRPRIKQLVDTLFKNVPSG
VGSQGRIKLHWTQIDDVLVDGAKWAVDNGYGWERDLERLEEGGRMEGADPEAVSQRAKQRGAP
QLGSLGSGNHFLEVQVVDKIFDPEVAKAYGLFEGQVVVMVHTGSRGLGHQVASDYLRIMERAIRK
YRIPWPDRELVSVPFQSEEGQRYFSAMKAAANFAWANRQMITHWVRESFQEVFKQDPEGDLGM
DIVYDVAHNIGKVEEHEVDGKRVKVIVHRKGATRAFPPGHEAVPRLYRDVGQPVLIPGSMGTASYIL
AGTEGAMKETFGSTCHGAGRVLSRKAATRQYRGDRIRQELLNRGIYVRAASMRVVAEEAPGAYK
NVDNVVKVVSEAGIAKLVARMRPIGVAKGAAALEH.
SEQ ID NO: 17 (RB69Rnl, derived from *Enterobacteria phage* RB69, Rnl1 family): 
MEKLYYNLLSLCKSSSDRKFFYSDDVSPIGKKYRIFSYNFASYSDWLLPDALECRGIMFEMDG
ETPLRIASRPMEKFFNLNENPFTLSIDLNDVKYLMTKEDGSLVSTYLDGNMVRFKGSIKSDQAASAT
 
SILLDINHKDLADRLLELCNDGFTANFEYVAPSNKIVLTYPEKRLILLNIRDNNTGKYIEYDDIYLDPVF
RKYLVDRFEAPEGDWVPGVKSSTNIEGYVAVMKDGSHFKLKTDWYVALHTTRDSISSPEKLFLAIM
NGASDDLKAMYADDEFSFKKVELFEKAYLDFLDRSFYICLDAYDKHKGKDRKTYAIEAQAICKGAQ
SPWLFGIIMNLYQGGSKEQMMTALESVFIKNHKNFIPEGY.
SEQ ID NO: 18 (RnIB-BRnl, derived from *Aeromonas virus* Aeh1, RnI2 family): 
MIVGRYEKQIRQLHKCLVHLDMIDKEDSIGVFGEYAGIMSYGKWIQTGIEYGPQDFYVFDIIVQP
LDGDQFMLEDLQVEELCKALGLKTAPLLKVGTFADIMKIPVDLQSVVMEVNAGKPIDIRVGTDNIAE
GYVAKPNVPARFKNGNRVAIKCKNEKWSETGKGRAVQVEVKELSEKDKALVLDITRYITDNRLKNV
LSKMDTPKTSEFGKVLGLMSKDVQKDYERDELDGVSVKEATDEAGRVMRMINTEIGNMIRPNWV
SICDGDW.
SEQ ID NO: 19 (UA1Rnl, derived from *Escherichia phage* UFV-AREG1, Rnl1 family): 
MQELFNNLMELCKDSQRKFFYSDDVSASGRTYRIFSYNYASYSDWLLPDALECRGIMFEMDG
EKPVRIASRPMEKFFNLNENPFTMNIDLNDVDYILTKEDGSLVSTYLDGDEILFKSKGSIKSEQALMA
NGILMNINHHQLRDRLKELAEDGFTANFEFVAPTNRIVLAYQEMKIILLNIRENETGEYISYDDIYKDA
ALRPYLVERYEIDSPKWVEEAKNAENIEGYVAVMKDGSHFKIKSDWYVSLHSTKSSLDNPEKLFKTI
IDGASDDLKAMYADDEYSYRKIEAFETTYLKYLDRALFLVLDCHNKHCGKDRKTYAMEAQGVAKGA
GMDHLFGIIMSLYQGYDSQEKVMCEIEQNFLKNYKKFIPEGY.
SEQ ID NO: 20 (KP27Rnl, derived from *Klebsiella phage* KP27, RnI2 family): 
MFKKYSSLTNHYEGKFINGVIMNGLTGGVVVAREKIHGANFSFITDDGITVTPAKRTDVVKPA
EDFYGCSAVVAKYSPGIRKMWETLKKTGTYDDLVIQVYGEFAGRGVQKDVDYGEKDFYVFDIRVN
GEFLPDNLCSLISRSHGLKMAPLLGYGTFEEIKELPITFESVVNKANSGIGSDNTVYGEFVYPIMDV
EEGNIAEGFVMKPVSPAFMPNGERVAIKCKTTKFTEKKAKKATRFNAPVSLSEKDKNQLDEFVCYL
TENRVKNVLSKLDLASITAKDFGRIMGLTVQDAIEEISRNHGPFLEQFEDPAMAKKLFVTEAQNMIR
PVWGKILNHEF.
SEQ ID NO: 21 (FpRnl, derived from *Fusarium proliferatum,* Rnl5 family): 
MPRKLVTVRHVSAITAIPRADRIAAATVDGWTCVVPTNIFKAGDRAVFFEIDSLLPATDPRFAPL
APKIIGPGGPTSAPDIRVQTIQIRGVLSQGLLLPLADFPEIVAKLDGIPSDKLRDISFEDILNVRKFEKP
AIPLHQTSTSDAPLPEYPDFIPRTNQERVQNLTDVFSEHGTEIFEESTKMDGSSTTVFYLNDGNPLA
KTVPSETRHNGVAVCSRNRILVENHPRSPPLFYATARALNLHEKLPKIGRNIALQGELCGSSIQSNF
EGFLKGTHCFFLFAVYDIDEQRYLPPREVYERWAPLLGVEHVPVHGYRPLNEMGSQITDLVDRAE
GRGINGRKREGIVFKREDGQFSFKAISNSYLLTHGE.
SEQ ID NO: 22 (TbRnl, derived from *Thermococcus barophilus,* Rnl1 family): 
MVSLHFKHILLKLGLDKERIEILEMKGGIVEDEFEGLRYLRFKDSAKGLRRGTVVFNESDIILGF
PHIKRVVHLRNGVKRIFKSKPFYVEEKVDGYNVRVAKVGEKILALTRGGFVCPFTTERIGDFINEQF
FKDHPNLILCGEMAGPESPYLVEGPPYVEEDIQFFLFDIQEKRTGRSIPVEERIKLAEEYGIQSVEIF
GLYSYEKIDELYELIERLSKEGREGVVMKSPDMKKIVKYVTPYANVNDIKIGSRIFFDLPHGYFMQRI
KRLAFYIAEKRIRREDFDEYAKALGKALLQPFVESIWDVAAGEMIAEIFTVRVKKIETAYKMVSHFER
MGLNIHIDDIEELGNGYWKITFKRVYDDATKEIRELWNGHAFVD.
SEQ ID NO: 23 (TbREL1Rnl, derived from *Trypanosoma brucei,* RnI2 family): 
MDQSDFSPYIEIDLPSESRIQSLHKSGLAAQEWVACEKVHGTNFGIYLINQGDHEVVRFAKRS
GIMDPNENFFGYHILIDEFTAQIRILNDLLKQKYGLSRVGRLVLNGELFGAKYKHPLVPKSEKWCTLP
NGKKFPIAGVQIQREPFPQYSPELHFFAFDIKYSVSGAEEDFVLLGYDEFVEFSSKVPNLLYARALV
RGTLDECLAFDVENFMTPLPALLGLGNYPLEGNLAEGVVIRHVRRGDPAVEKHNVSTIIKLRCSSF
MELKHPGKQKELKETFIDTVRSGALRRVRGNVTVISDSMLPQVEAAANDLLLNNVSDGRLSNVLS
KIGREPLLSGEVSQVDVVLMLAKDALKDFLKEVDSLVLNTTLAFRKLLITNVYFESKRLVEQKWKEL
MQEEAAAQSEAIPPLSPAAPTKGE.
SEQ ID NO: 24 (TbREL2Rnl, derived from *Trypanosoma brucei* gambiense DAL972, RnI2 family): 
MVGGDGSIFERYTEIDNSNERRINALKGCGMFEDEWIATEKVHGANFGIYSIEGEKMIRYAKR
SGIMPPNEHFFGYHILIPELQRYVTSIREMLCEKQKKKLHVVLINGELFGGKYDHPSVPKTRKTVMV
AGKPRTISAVQTDSFPQYSPDLHFYAFDIKYKETEGGDYTTLVYDEAIELFQRVPGLLYARAVIRGP
MSKVAAFDVERFVTTIPPLVGMGNYPLTGNWAEGLVVKHSRLGMAGFDPKGPTVLKFKCTAFQEI
STDRAQGPRVDEMRNVRRDSINRAGVQLPDLESIVQDPIQLEASKLLLNHVCENRLKNVLSKIGTE
PFEKEEMTPDQLATLLAKDALKDFLKDTEPSIVNIPVLIRKDLTRYVIFESRRLVCSQWKDILKRQSP
DFSE.
SEQ ID NO: 25 (JS98Rnl, derived from *Escherichia phage* JS98, RnI2 family): 
MFKKYSSLENHYNSKFIEKLRTNGLTGGEWVAREKIHGTNFSLIIERDAVTCAKRTGPILPAEDF
YGYEIVLKNYADSIKSVQHLIESINYQSYQIYGELAGPGIQKNVDYGDKDFYVFDIRVTKEDGTESVL
TDTLMEAFCIIHKFKVAPCLATGSFEDLIKLPNDFDSVIPDYNFAVDNAGLTIANSTDFIPKVEGKVFT
AEGFVLKPDIPTWLPNGNRVAIKCKNSKFSEKKKSDKPIKAAVVLSQDDMDLMWQFTDYVTVNRIN
NVISKIGEVSKKDFGKVMGLTVQDILEEAAREELELTDAENPVEVKKQLVECVKDTLRAVWIELVS.
SEQ ID NO: 26 (VgRnl, derived from *Variovorax gossypii,* Rnl5 family): 
MVPGLFSFCVTINHREKTTMRKLVTVRTVDFVRPIPGADAIECAVVEGWSVVIKKGEFAVGDR
CVFFEIDAFLPLDDPRFAFLEKAAITWNEQRGVRLRTMKLRGQISQGLILPLSQFPEIDALALADPDL
RHRDWAELLGIGKWEPVIPACLSGEVEGPFPSFISKTDQERIQNLPEVLAANDGLAFEVTVKLDGS
SMTVFHNAGAVGVCGRNWQLRETPGNSLWRVAREDRLLEVLATLGRNLALQGEIIGEGIQGNPEK
LRGQQFHVFDVFDIDRGAYCGMDERNAVIDALRVLGATVRSVPLLEVAALDRFDGSMAGVLAYAE
GPSLNPGTSREGVVFKRLDGGLSFKAISNTYLLKHADR.
SEQ ID NO: 27 (UpRnl, derived from *Undibacterium pigrum*, Rnl5 family): 
MRKLVTIRTVSNIRPIPDADAIECASVDGWSVVVKKNEFQVGDACVYFEIDSFLPLSDARFAFL
EKNKIIWNEKNGIRLRTIKLRGQISQGMILPLNQFPELIELTDDGNADFSQVLGIEKWEPAIPASLSGE
VEGAFPEYIRKTDQERIQNLTEVLSTQAQEEFEVTIKLDGSSMTVFHNAGVSGVCGRNWWIRESE
ANSLWRVAKQNQMLTALTAFGRNLALQGEIIGEGIQGNPEKMRGQDFYLFDIFDIDASRYLGRAER
QEVLAALRALGATVHEVPWLENMTLERFAGDVSKVLDYAEGGSLNPATAREGVVFKRLDGSMSFK
AISNTYLLKHGDR.
SEQ ID NO: 28 (PpPRnl, derived from *Panteoa phage* Phynn, Rnl5 family): 
MFVKYSSLTNHYEGKFINGIVMNGLASGEWVAREKIHGANFSFLTCDGATVIPAKRSGEILPGE
RFYDCENVVAKYLESVRNLWQKLFVTGFYDVLNVQIFGELAGRGVQKDVDYGEKDFYVFDIKVNG
EYLDDRLVSSLARECGLKMAPLLGYGSYEHLKELPLTFESVVLFSPSRTEVKGEETVHNINEAADD
VENIAEGYVLKPVVTAYMTNGSRVAIKCKTSKFSEKKNKSTTAFNAPVSLSEADKLKLGEYVNYLTE
NRVKNVLSKIDASNLTAKDFGRVAGLTVQDALEEIERNEGDFVTKFENPAFAKKLFVSEAQSLIRPV
WGLILNNEF.
SEQ ID NO: 29 (SpMRnl, derived from *Stenotrophomonas phage* Marzo, Rnl1 family): 
MYLNLQMMRDALGHDETVRFKEETVDGREFVIVSYMIANDDLWSRSFGAEARGITFDKVTGE
CVSRPFHKFFNVGEKEFTQPVALQNTVWTAYPKIDGSMITPVMVNGKVRLKTKKSFYSNVAREAQ
ANLDPETEGQMAAFLISGWTPIYEYTSPTTKIVVQHDKPAFKLLAGRATQDGHYLTPTDLALHPDAI
KPEFHSASINELLYTARDLEGVEGWVLVDHEGGYDRVKIKTDWYNRLHRVLDLRVRDVVEFIRDE
KLDDMIPNLLNMGVDMDVIQSIENRVVNAISDAVTHVKTVVLGASGLPLKEAVQFIEKHGGIYVKAC
HRTWRTGCIEPQMELIVRLYFQYHLQDYELTSIGNPNFGGKDG.
SEQ ID NO: 30 (WaRnl, derived from *Waterburya agarophytonicola,* Rnl1 family): 
MELQDYLRNRGLDKLTEEYNIKVNRHSKIDNLVCLKYSQLESPMGEKIVQQCRGIIFDETNNW
NIVSYPYDKFFNYGESYAPKLNWDAARIYEKLDGSLMTLFYYEGEWRVQSSGMADAGGDVSGFK
YTFQSLFWKVWQELDYQLPTETEYCFMFELMTPYNRIVVRHDRHKLVLHGVRNKVTLKEEDPQIW
 
TDKYNWQLVATYPLQTLEEIVAMTDKLDPMDSEGYIICDREFKRIKVKSPQYVAISHLKTGFSSRRM
LEIVVTNEGEEFLNYYPEWQELYQQIATQYNALIEEIEEQYYKYQNIPVQKDFAIAVKNLSYSGILFA
LRAGKSNSVKESLAQTSIYKLENLLNINFQELG.
SEQ ID NO: 31 (SavRnl, derived from *Streptomyces avermitilis,* RnI2 family): 
MSTLRVTAEVLTIHPHPNADALELAQVGLYRAVVAKGAYRTGETAVYIPEQSVLPAGLIEELGLT
GRLAGSGSDRVKAVRLRGELSQGIVCRPKALADVDLARTVADGTDFAELLGITKWVPPIPPTMSGE
IESVPDLLRWVDIENIQRYPDIFTPGEPVVLTEKLHGTACLVTYLADEDRVHVSSKGFGAKSLALKE
DPRNLYWRAVHGHGVAQAAARLAERLGARRVGIFGEVYGAGVQDLTYGADGRRDTLGYAVFDVS
ADIDGEVRWLDAADRLDGELPLVPRLYEGPYDIERVLETASGRETVSGHGLHLREGVVIRSATERR
SPVTGGRAIAKAVSPAYLTRKGGTEYE.
SEQ ID NO: 32 (P000VRnl, derived from *Escherichia phage* p000v, RnI2 family): 
MEKLYYNLLSLCKSSSDRKFFYSDDVSPIGKKYRIFSYNFASYSDWLLPDALECRGIMFEMDG
ETPLRIASRPMEKFFNLNENPFTLSINLDDVKYLMTKEDGSLVSTYLDGGTVRFKSKGSIKSDQAVS
ATSILLDIDHKNLADRLLELCNDGFTANFEYVAPTNKIVLSYPEKRLILLNIRDNNTGEYIEYDDIYLDP
VFRKYLVDRFEVPEGDWTSDVKSSTNIEGYVAVMKDGSHFKLKTDWYVALHTTRDSISSPEKLFLA
IVNGASDDLKAMYADDEFSFKKVELFEKAYLDFLDRSFYICLDTYDKHKGKDRKTYAIEAQAVCKGA
QTPWLFGIIMNLYQGGSKEQMMTALESVFIKNHKNFIPEGY.
SEQ ID NO: 33 (FsRnl, derived from *Fusarium subglutinans,* Rnl5 family): 
MPRKLVTVRHVSAITAVPRADRIAAATVDGWTWVPVNVFQVGDRAVFFEIDSLLPATDPRFA
PLAPKIIGPGGPTSAPDIKFGGFAELDGIPSYKLRDISFEDILHVRKFEKPAMPLQQTSASETPLPEY
PDFIPRTNQERVQNLTDVFSEHGTEIFEESTKMDGSSMTVFYLNDANPLANTVPSEARHNGVSVC
SRNRILVENHPRSPSLFYSTARALNLHETLPKIRRNIALQGELCGSSIQSNFEGFPKGTHCFFLFAVY
DIDEQRYLPPREVYETWAPLLGVKHVPVHGYRPLNEMGSQITDLVNRAEGRGINGRKREGIVFKR
EDGQFNFKAISNSYLLTHGE.
SEQ ID NO: 34 (JN02Rnl, derived from *Vibrio phage* JN02, RnI2 family): 
MEKLYYNLLSLCKSSSDRKFFYSDDVSPIGKKYRIFSYNFASYSDWLLPDALECRGIMFEMDG
ETPVRIASRPMEKFFNLNENPFTLSINLDDVKYLMTKEDGSLVSTYLDGGTVRFKSKGSIKSDQAVS
ATSILLDIDHKNLADRLLELCNDGFTANFEYVAPTNKIVLTYPEKRLILLNIRDNNTGEYIEYDDIYLDP
VFRKYLVDRFEVPEGDWTSDVKSSTNIEGYVAVMKDGSHFKLKTDWYVALHTTRDSISSPEKLFLA
IVNGASDDLKAMYADDEFSFKKVELFEKAYLDFLDRSFYICLDTYDKHGKDRKTYAIEAQAVCKGA
QTPWLFGIIMNLYQGGSKEQMMTALESVFIKNHKNFIPEGY.
SEQ ID NO: 35 (SsRnl, derived from *Shigella sonnei,* RnI2 family): 
MFKKYSSLENHYNSKFIEKLYTNGLTTGVVVAREKIHGTNFSLIIERDNVTCAKRTGPILPAEDF
YGYEIVLKKYDKTIKAVQEFMYTARAVSYQVFGEFAGGGIQKGVDYGEKDFYVFDILINTESGDNTY
LTDYEMQDFCNEFGFKMAPMLGRGTFDALIMIPNDLDSVLAAYNATASEDLVEANNCVFDANVIGD
NTAEGYVLKPCFPKWLPNGTRVAIKCKNSKFSEKKKSDKPVKTQVPLTEIDKNLLDVLACYVTLNRI
NNVISKIGAVTPKDFGKVMGLTVQDILEETSREGIVLTSSDNPNLVKKELVRMVQDVLRPAWIELVS.
SEQ ID NO: 36 (ToRnl, derived from *Thelonectria olida,* Rnl5 family): 
MARKLVTVRRIAALNEIPGADRIEAASVDGWTCVVPVGQFEKGGLAVFFEIDSLLPAKDERFAA
FAKTNDGEDIRLKTVRIRKVTSQGLLMPLATFPEIKAVVDKLALELQPAEVEARLRAVSFEKELGVRK
FVAPEQASQKGVAGGRPAYMADFPVFIPRTDQERIQNMPGAFDEWPDSVFQETTKMDGSSMTAY
FLRRDSPYFDMLPPLPLTAKNAEFPNGRFGVCSRNRDIGEVPNSGLFWPTALANNLPSTLSRLDR
NIALQGELCGSSIQANFEGFAPGAHDFFLFTIWDVDKQRPLPPREVHEIWAPKLGVKHVPVVGYHA
LKYVASSVGELVKRADGKGINGKKREGIVLKHVDGKVSFKAISNSYLLKHGE.
SEQ ID NO: 37 (CpMRnl, derived from *Citrobacter phage* Merlin, RnI2 family): 
MFEKYSTLENHYNNKFIERIRSAGFDLTETWVAREKIHGTNFSIIITKDTVTCAKRTGPILEAEDF
FGYEIILKKYDKSIKALQDTMKNMTTESYQLFGEFAGGGIQKGVNYGEKDFYVFDCLVKTPGGIVE
YSDDYILTAFCNVFGFKMAPLLGRGKFDDLIQMSNMLDVVVNDYNKLAEADLEAANLKVWPVVVS
EDNIAEGYVLKPCYPKFFNNGARVAIKCKNSKFSEKSKSDKLIKAKVELTEADKKCLSAFSEYVTIN
RVNNVISKIGTVTTKDFGRVLGLTMKDILEEAAREEVVLTSADNPDIVKKELTRILQETLRPAWIELIS.
SEQ ID NO: 38 (FaRnl, derived from *Fusarium albosuccineum,* Rnl5 family): 
MPRKLVTVRQISAISPITGADRIETATIDGWTCVVPVGDFKPGDRGLYFEIDSLLPGADPRWAF
LAPKNGPTPPADIRVKTLRVRGVLSQGLLLPLLSFPEVTAVIDALSPAEIDVKLRDMSFEEVLRVRKY
EKPVVPLPNGAPDVTPLPEFPYFVPKTEAERVQNLPDVFISHGDEVFQESTKMDGSSMTVFYLGA
DSPHHDILDADISSDGVYVCSRNRMLVENHPRSPPLFWSTARALNLPATLSSLGRNIAIQGELCGS
SIQSNYEGFGRGEHNFFLFSVWDIDAQRYLPPREVHETWAPRLGVKHVPVHGYRPLKEIGGSVAD
LVARAEGKGVDGRKREGIVLKQEDGSLSFKAISNSYLLKHGE.
SEQ ID NO: 39 (RnIARnl, derived from *Vibrio phage* nt-1, Rnl1 family): 
MSFVKYTSLENSYRQAFVDKCDMLGVRDWVALEKIHGANFSFIVEFDGGYTVTPAKRTSIIGA
TATGDYDFYGCTSVVEAHKEKVELVANFLWLNEYINLYEPIIIYGELAGKGIQKEVNYGDKDFWAFD
IFLPQREEFVDWDTCVAAFTNAEIKYTKELARGTLDELLRIDPLFKSLHTPAEHEGDNVAEGFVVKQ
 
LHSEKRLQSGSRAILKVKNEKFKEKKKEGKTPTKLVLTPEQEKLHAEFSCYLTENRLKNVLSKLG
TVNQKQFGMISGLFVKDAKDEFERDELNEVAIDRDDWNAIRRSLTNIANEILRKNWLNILDGNF.
SEQ ID NO: 40 (SH7Rnl, derived from *Shigella phage* SH7, Rn2 family): 
MFKKYSSLENHYNSKFIEKLYSLGLTGGEWVAREKIHGTNFSLIIERDKVTCAKRTGPILPAED
FFGYEIILKNYDDSIKAVQDIMETSAVVSYQVFGEFAGPGIQKNVDYGDKDFYVFDIIVTMESGDVT
YVDDYMMESFCNTFKFKIAPLLGRGKFEELIKLPNDLDSVVQDYNFTVDHAGLVDANKCVWNAEA
KGEVFTAEGYVLKPCYPSWMPNGNRVAIKCKNSKFSEKKKSDKPIKVKVELSEADNKLVGILACYV
TLNRVNNVISKIGEIGPKDFGKVMGLTVQDILEETSREGITLTQADNPSLIKKELVKMVQDVLRPAWI
ELVS.
SEQ ID NO: 41 (ST2Rnl, derived from *Vibrio phage phi-ST2,* Rnl1 family): 
MSFVKYTSLENSYRQAFVDKCDMLGVKEWVALEKIHGANFSFIVEFKPSTEEVPGEMSVTPA
KRTSTIGANAMGDYDFYGCTSVVEAHIEKMQDISNWLFANDFIKNDETIIVYGELAGKGIQKEVNYG
DKDFWAYDILCPETGEFLDWDVVLKACKFAGVKTTHEIARGTLDELLKIDPLFRSFHTPADVDSEN
VAEGFVVKQLKAEKRLHNGSRAILKVKNEKFKEKKNKQGKTPRAKVVLTEEQEKLHAAFSCYLTE
NRLRNVLSKIGKVEAKQFGMVSGLFVKDAKDEFERDERDEVAIPRDDWDVIKRSLVNVANEILRKN
WLNIVDGTF.
SEQ ID NO: 42 (KP15Rnl, derived from *Klebsiella phage* KP15, RnI2 family): 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
EFLPDNLCSLISRSHGLKMAPLLGYGTFEEIKELPITFESVVNKANSGIGSDNTVYGEFVYPIMDVEE
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFIEQFEDPAMAKKLFVTEAQNMIRPV
WGKILNHEF.
SEQ ID NO: 43 (JN02Rnl, derived from *Escherichia phage* JN02, RnI2 family): 
MFKKYSSLENHYNSKFIEKLYTNGLTTGVWVAREKIHGTNFSLIIERDNVTCAKRTGPILPAED
FYGYEIVLKKYDKAIKAVQEVMESISTSVPVSYQVFGEFAGGGIQKGVDYGEKDFYVFDIIINTESDD
TYYMSDYEMQDFCNTFGFKMAPMLGRGTFDSLIMIPNDLDSVLAAYNSTASEDLVEANNCVFDAN
VIGDNTAEGYVLKPCFPKWLSNGTRVAIKCKNSKFSEKKKSDKPVKTQVPLTEIDKNLLDVLACYV
TLNRVNNVISKIGTVTPKDFGKVMGLTVQDILEETSREGIVLTSSDNPNLVKKELVRMVQDVLRPA
WIELVS.
SEQ ID NO: 44 (YpRnl, derived from *Yersinia phage* JC221, RnI2 family): 
MFKKYSSLTNHYEGKFINGVIMNGLTGGVWAREKIHGANFSLMTKDGINVIPAKRSGEILPAE
QFYGCEPVVAKYSPAIRMIWRWIHDLNVINDAHEDLEIQVYGEFAGRGVQKDVDYGEKDFYVFDIR
VNGLFLADNVVANYATGSGLKMAPLLAYGTFEEIKELPITFDSVVNLANSGMVMKSNDFEPEFKNY
MTLKDGEGVNIAEGFVMKPVIPAFMPNGDRVAIKCKTTKFTEKKNKQANRFNAPVALSENDKAKLE
VFTCYITENRVKNVLSKMDRTNLTAKDFGRVMGLTVQDALEEIERNYGPFLEQFEDPTLAKKTFSN
EASNLVRENWGAILNNEF.
SEQ ID NO: 45 (CpRnl, *Cronobacter phage* vB_CsaM_GAP161, RnI2 family): 
MFVKYSSLTNHYEGKFINGVIMNGFTGCVWVAREKIHGANFSLITSDGIKVIPAKRSGEILPAEQ
FYGCEPVVAKYSEPVRKLWEILANACEYKEYADTGSLVVQVYGEFAGRGVQKDVDYGEKDFYVFD
IRVNGEFLPDNVVATYSVAVGLKMAPLLAYGSFDEIRALPITFDSVVNLANSGAIPAKNGVEPEFKNF
MTLKDGEGENIAEGFVMKPVQPAFMPNGERVAIKCKTTKFTEKKNKQANRFNAPSELSETDKAKL
NEFTCFLTENRVKNVLSKIDSANLTAKDFGRVMGLTVQDALEEIERNYGPFLEQFENPTLAKKTFTN
EASNLVRENWGAILNNEF.
SEQ ID NO: 46 (AmeRnl, derived from *Amsacta moorei entomopoxviru*, Rnl5 family): 
MVYIILDVGSKAKGYDIISSDYDYIIITKSSADIYAEEWIEDNKRLINRHSNKKNNNDITFVDISKAF
MGIIKGNYYFLGIYGTKENIKNDMIFYFVRYITKYYISLILKTMTKYQINNTSDREPKNLLALLYNLSYV
NYWLKNNDFPVINKLPELLEEDKIELYNILMMKRLNNELATEEQGSIIQDYRKVLLENVNKLEKPKKI
KNISNIIMMYLICNKPLYIPEILNNDDEINKIIYPSIKQLNHCKNSLLYGKEIFVQEKLDGCNFRIIYNNGII
TFGSRYTYYENKNFMNYYRIKDKLIDCTNKINKFLNLKKFIIYGELIGSYQNDEGINKLIIKNVNYFND
NRIEYYAYEIKNCDNKNKIHFIDFDICQIVLNAAGFNVINYETIKYNDFIENIKYKSIVFNHNDESKVEG
YIIRYKNIRYKVKKSYNLTKLTHKNILYNITEESVLNFINNIDINQDNVLYYITKYYKNLIHMNNIDNNVPI
YKIFGKIYGFINKKYKFNDNNIYKNKLSEFYKLL.
SEQ ID NO: 47 (CsRnl, derived from *Chitinophaga sp.* S165, Rnl5 family): 
MRRLSIYLASIDEINQIRNPKGFTFRSKEMERKLVSPVVIDDIQPIVGADLIEVATVKGWKLVVKKN
EFSIGDHAVYCEIDSFLPIREEFEFLRKSSYRKMGEQEGFRLKTMKLRGQISQGLLLPVSLLEGYTY
QIGEDVSARLGVIKYEPPVPAALGGIAKGLFPSFMPKTDEERIQNLSGQYEEFRKHTFYVTEKLDG
SSATFYLNEGVFGVCSRNLDLEETEDNTFWKIARVEKLEEKLAALGKNFAIQGELIGEGVQGNAYG
LRGQTVRFFNAFDIDRYEYLGLDEFKALFASLGLQTVPILDEEYVLPASLDEMLTIADGASVLSPKGK
EVEREGLVIRSKDRKISFKVISNKFLLNER.
SEQ ID NO: 48 (CAbRnl, derived from *Candidatus Aminicenantes bacterium,* Rnl5 family): 
MERKLATIRKVNEIKPIENADMIELAIVDGWQCVVKKGEFKPGDLGVYFEIDSYLPIEERYEFLR
KSSYKKIPDQLQGQRAEGFRLKTVKLRGQISQGLMLPLDMFPELKTTSPGSDVTESLNVELYEPPV
PANLAGQVRGKLPGFLKKTDEERIQNLPEYFENYKNISFECTEKIDGSSMTVYSTEDDQGVCSRNL
NLKEDEQNTLWKIAKQLELHKLLKELNKNLALQGEIAGEGIQKNPLKIKGQHLFLFNIWDIEKRQHLT
PEERMEIFDSFEKRVPIRHVPVISSEIKVFEKYKTMQELLDFAVGKSLLNKDIGREGIVLKSLESIGRE
IISFKVISNKYLLKHNA.
SEQ ID NO: 49 (MrRnl, derived from *Massilia rubra,* Rnl5 family): 
MRKLATVRKVIDIAPIPGADAIECVTVDGWKVVSKKGEFQIGGNALYLEVDSWVPEALAPFLCKD
KREFNGVSGARLRTVKLRGQISQGLLLPVPEGAAEGDDLSEMLGIQKYEPPIPADLQGIVKGPFPS
FVRKTNQERIQNLVAELEEWVEAGLEWEVTEKLDGSSLTAYLFQGEFGVCSRNLDLLETENNSYW
KLARENKLEELLRASGRNLAVQGELIGPNVQGNPYGVGAPQLHVFDVYDIDRGGYLTSAERMALL
DGAGILHCPVLGTQHLAGASVASLLASAEGKSALKAGTEREGFVFKCLSADVSFKAISNKFLLGEK.
SEQ ID NO: 50 (lbRnl, derived from *Ignavibacteriaceae bacterium,* RnI5 family): 
MRKLATIQIVSDVQPIEGADKIELASILGWKVVIKKGEFEPGDPAIYCEVDSLLPVKPEFEFLRKSS
FKRMSDGKEGFRLKTIKLRGQISQGLLLPITALGAHVIDLVPGTDVTNLLEIVKYETPIPAKLAGEVKG
PFPGFLSKTDEERVQNLLDELPLYKNSLFYVTEKVDGTSITIYLKDGQFGICSRNLELVENPENTYW
KVVRELDIENKLRNLGRNIALQGELLGEGIQKNIYKFKGQTIKFFDAFDIDLYRYFDRKSFFEIMNNF
GLETVPVVFDDFTLPESIDEILQMANGKSQLNPDANREGLVFRTTDDKRFSFKAISNNFLLLDEE.
SEQ ID NO: 51 (TaRnl, derived from *Thermoprotei archaeon,* RnI3 family): 
MVPPLKRISEFVWEIPQKYKREMRVPARIFSDEVLLEKMKSDLTLEQAANVACLPGIYKYSIVLP
DGHQGYGFPIGGVAAIDYEEGVVSPGGVGYDINCGVRLLRTDLSYEDVKPKIKELINTLFRMVPSG
LGSRSHLRLSYGELDEVLASGVEWAVAHGYGWEEDLDRIEERGSMKLADPDKVSDTAKRRGSAQ
LGTLGSGNHFLEVQVVDKIYNPEVAKVFGITHEGQITVMIHTGSRGLGHQVCSDYLRIMERAVRKY
GIRLPDRELAAVPVKSKEGEDYLAAMAAAANFAWTNRQMIMHWVREAFRKVFGQSPEDLGMHLV
YDVAHNIAKIEEHTIDGGTRKVVVHRKGATRAFPRGHPAIPAIYRSVGQPVLIPGSMGTASWVLVGT
EAAMKITFGSTAHGAGRYLSRAAAVRTWRPRDVAAELERKGVVLRAANARVIAEEAPGAYKDVDR
VVEVSHRVGIATKVARLVPIGVTKG.
SEQ ID NO: 52 (KpRnl, derived from *Klebsiella pneumoniae,* RnI1 family): 
MLELYKNLMNLCESSEVAKFFYKDFTGPMDGKFRVFSYHYASYSEWLKPDALECRGIMFEMD
GDTPIRVASRPMEKFFNLNENPLTMGIDISDVEYIMDKADGSLVSSYVDDGYLYLKSKTSLYSDQAR
QASALLNSEEYSSLHQVILELALDGYTVNMEFVSPNNRVVLAYQEPQLFVLNVRNNATGEYIKYDD
 
LYANAKIRPYLINAYGISDPTTWVEGVRELEGVEGYIAVLNTGQRFKVKTEWYSALHHTKDSITSNE
RLFASVVSANSDDLRSLFAGDEYAIKKISAFEQAYLDYLGKSLELCQSFYDEYRGRARKDYAIAAQK
ATVNQRHLFGVIMNMYEGTVDVDKLLKDLERVFLKYWAGYVPKEYEKEIELSEE.
SEQ ID NO: 53 (AbRnl, derived from *Acidobacteria bacterium,* Rnl1 family): 
MKQMYDNLKALTQKNDMFFSSIQTTPSNKPVEIFSYHIASYSDWLEPDAIECRGIMFDITDKEN
PVILSRPMEKFFNLGENPLALPEDVLPLVKTIEEKRDGSLISTYLDDGKLYTKSKGSLYSDQANDSN
RFLMRSENKDFKQALKTLAELGYTANMEYTSPKNQIVLKYEKQELIVLNIRNTETGDYLTLDDLAEL
DAENPEHTIYTHIAERFVDYEEVDGMSDEDVERIRDMKGIEGYVLVGQNQRVKLKTDWYVNLHRL
KDNINNNRRLVESVVESRTDDLRQLFEHDQGSIDKIKEFEQYVLSKIKSCLAEVRTVYERVKHLDRK
HYAINAQRYVDYTPLFSVIMRQFDDYNEENVVENIKQIALKNVDDFIPMHYK.
SEQ ID NO: 54 (SeRnl, derived from *Salmonella enterica,* RnI2 family): 
MFKKYSSLENHYNSKFIEKLYSLGLTGGEWVAREKIHGTNFSLIIERDKVTCAKRTGPILPAEDF
FGYEIILKNYADSIKAVQDIMETSAVVTYQVFGEFAGPGIQKNVDYGDKDFYVFDIIVTTESGDVTYV
DDYMMESFCNTFKFKMAPLLGRGKFEELIKLPNDLDSVVQDYNFTVDHAGLVDANKCVWNAEAK
GEVFTAEGYVLKPCYPSWLPNRNRVAIKCKNSKFSEKKKSDKPIKTKVELSEADNKLMGILACYVTL
NRVNNVISKIGEIGPKDFGKVMGLTVQDILEETSREGITLTQADNPSLIKKELVKMVQDVLRPAWIEL
VS.
SEQ ID NO: 55 (YpRnl, derived from *Yersinia phage* vB_YepM_ZN18, RnI2 family):

MFKKYSSLENHYNSKFIEKLYSLGLTGGEWVAREKIHGTNFSLIIERDKVTCAKRTGPILPAEDF
FGYEIILKNYEDSIKAVQDIMETSAVVSYQVFGEFAGPGIQKNVDYGDKDFYVFDIIVTTESGDVTYV
DDYMMESFCNTFKFKMAPLLGRGKFEELIKLPNDLDSVVQDYNFTVDHAGLVDANKCVWKAEAK
GEVFTAEGYVLKPCYPSWLHNGNRVAIKCKNSKFSEKKKSDKPIKAKVELSEADNKLVGILACYVTL
NRVNNVISKIGEIGPKDFGKVMGLTVQDILEETSREGITLTQADNPSLVKKELVKMVQDVLRPAWIEL
VS.

### Embodiment 2

The present application designed a plurality of non-natural RNA substrates.
P8 (4 nt):mA-fC-mG-fG,
P9 (5 nt):mG-fG-mU-fC-mA,
P10 (6 nt):fC-mU-fG-mA-fG-mU,
The expected length of a target product P13 (SEQ ID NO: 56) was 15 nt (the molecular weight was 5037);

The length of a RNA splint (single stranded RNA template) P14 (SEQ ID NO: 57) was 15 nt (the molecular weight was 4917).

P13 (15 nt): mA-fC-mG-fG-mG-fG-mU-fC-mA-fC-mU-fG-mA-fG-mU (SEQ ID NO: 56).

P14 (15 nt): mA-fC-mU-mC-mA-fG-fU-mG-mA-fC-fC-mC-fC-mG-mU (SEQ ID NO: 57).

m represented 2'-OCH₃ modification, and f represented 2'-F modification.

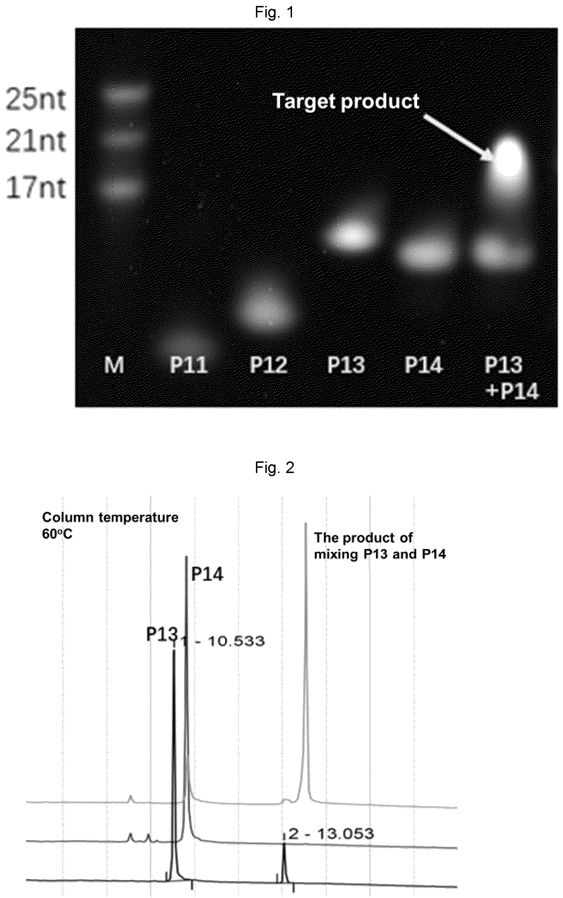

Firstly, an analysis method was developed for the target product. The analytical method for the target product included denaturing urea-polyacrylamide gel electrophoresis (PAGE) and ultra performance liquid chromatography (UPLC). The PAGE analysis result showed that the target product P13 might form a tightly bound double stranded product after being mixed with the single stranded RNA template P14, as shown in Fig. 1. The UPLC analysis result also confirmed the presence of the double stranded product, as shown in Fig. 2.

### Embodiment 3

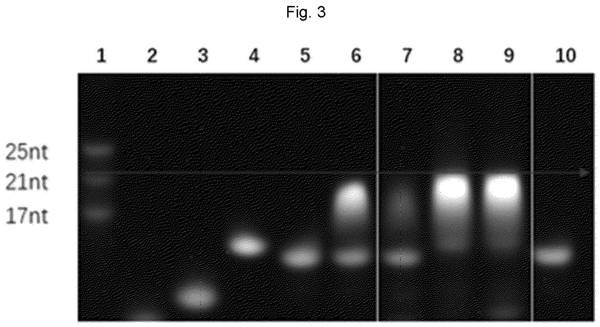

Specific ligation of a plurality of non-natural RNA fragments was achieved with non-natural single stranded RNA templates. The experimental content included that annealing of P8, P9, P10, and P14 was performed firstly so that they were combined together, then an expression-purified RNA ligase was used to perform the ligation of two nicks. The first attempt was a 10 µL reaction system, and the reaction conditions included substrate concentrations (P8, P9, P10, and P14) of 20 µM, enzyme amount of 0.2 mg/mL, ATP 1 mM, ligase reaction buffer (T4 DNA Ligase Reaction Buffer (10×), NEB, item number B0202S), reaction temperature of 16 °C, and reaction time of 16 h. After the reaction was ended, after high-temperature treatment and centrifugation, a supernatant of the reaction system was taken for denaturing gel electrophoresis analysis. The results were summarized in Table 1 below, and all 55 RNA ligases showed ligation activity. NgrRnl and JN02Rnl with the higher catalytic activity were selected for a 50 µL reaction to verify the reaction. The denaturing gel electrophoresis analysis results showed that NgrRnl and JN02Rnl enzymes were reacted to generate target products with high purity (as shown in Fig. 3). In Fig. 3, Lane 1 was ssRNA ladder, Lane 2 was P11, Lane 3 was P12, Lane 4 was P13, Lane 5 was P14, Lane 6 was P13+P14, Lane 7 was an MthRnl reaction system, Lane 8 is a NgrRnl reaction system, Lane 9 was a JN02Rnl reaction system, and Lane 10 was a reaction system without a ligase added. At the same time, the mass spectrometry analysis results showed that the molecular weight of the product shown in the denaturing gel electrophoresis analysis was consistent with the expected molecular weight of the target product, this fully proved the generation of the target product.

**Table 1: Denaturing gel electrophoresis analysis results of system after reaction**

| SEQ ID NO: | Protein name | Protein derivation | Protein family | Ligation activity |
|---|---|---|---|---|
| 1 | DpRnl | *Diplonema papillatum* | Rnl2 | + |
| 2 | ACNPVRnl | *Autographa californica nucleopolyhedrovirus* | Rnl2 | ++ |
| 3 | SfRnl | *Shigella flexneri* | Rnl2 | +++ |
| 4 | MthRnl | *Methanothermobacter thermautotrophicus str.* Delta H | Rnl3 | + |
| 5 | Pab1020Rnl | *Pyrococcus abyssi* | Rnl3 | + |
| 6 | NgrRnl | *Naegleria gruberi* | Rnl5 | +++ |
| 7 | DraRnl | *Deinococcus radiodurans* | Rnl5 | + |
| 8 | DdRnl | *Dictyostelium discoideum* AX4 | Rnl5 | ++ |
| 9 | GzRnl | *Gibberella zeae* | Rnl5 | + |
| 10 | NcRnl | *Neurospora crassa* | Rnl5 | ++ |
| 11 | PoRnl | *Pyricularia oryzae* 70-15 | Rnl5 | + |
| 12 | *SppRnl* | *Shigella phage* pSs-1 | Rnl2 | ++ |
| 13 | *BpRnl* | *Buttiauxella phage* vB_ButM_GuL6 | Rnl2 | + |
| 14 | TbgRnl | *Trypanosoma brucei gambiense* DAL972 | Rnl2 | ++ |
| 15 | F48Rnl | *Klebsiella phage* vB_Kpn_F48 | Rnl1 | ++ |
| 16 | PhRnl | *Pyrococcus horikoshii* OT3 | Rnl3 | + |
| 17 | RB69Rnl | *Enterobacteria phage* RB69 | Rnl1 | ++ |
| 18 | RnlB-BRnl | *Aeromonas virus* Aeh1 | Rnl2 | +++ |
| 19 | UA1Rnl | *Escherichia phage* UFV-AREG1 | Rnl1 | + |
| 20 | KP27Rnl | *Klebsiella phage* KP27 | Rnl2 | +++ |
| 21 | FpRnl | *Fusarium proliferatum* | Rnl5 | ++ |
| 22 | TbRnl | *Thermococcus barophilus* | Rnl1 | ++ |
| 23 | TbREL1Rnl | *Trypanosoma brucei* | Rnl2 | + |
| 24 | TbREL2Rnl | *Trypanosoma brucei* gambiense DAL972 | Rnl2 | ++ |
| 25 | JS98Rnl | *Escherichia phage* JS98 | Rnl2 | ++ |
| 26 | VgRnl | *Variovorax gossypii* | Rnl5 | ++ |
| 27 | UpRnl | *Undibacterium pigrum* | Rnl5 | + |
| 28 | PpPRnl | *Panteoa phage* Phynn | Rnl5 | ++ |
| 29 | *SpMRnl* | *Stenotrophomonas phage* Marzo | Rnl1 | + |
| 30 | *WaRnl* | *Waterburya agarophytonicola* | Rnl1 | +++ |
| 31 | SavRnl | *Streptomyces avermitilis* | Rnl2 | +++ |
| 32 | P000VRnl | *Escherichia phage* p000v | Rnl2 | ++ |
| 33 | FsRnl | *Fusarium subglutinans* | Rnl5 | + |
| 34 | JN02Rnl | *Vibrio phage* JN02 | Rnl2 | +++ |
| 35 | SsRnl | *Shigella sonnei* | Rnl2 | ++ |
| 36 | ToRnl | *Thelonectria olida* | Rnl5 | ++ |
| 37 | CpM Rnl | *Citrobacter phage* Merlin | Rnl2 | +++ |
| 38 | FaRnl | *Fusarium albosuccineum* | Rnl5 | +++ |
| 39 | RnlARnl | *Vibrio phage* nt-1 | Rnl1 | +++ |
| 40 | SH7Rnl | *Shigella phage* SH7 | Rnl2 | ++ |
| 41 | ST2Rnl | *Vibrio phage phi-ST2* | Rnl1 | +++ |
| 42 | KP15Rnl | *Klebsiella phage* KP15 | Rnl2 | +++ |
| 43 | JN02Rnl | *Escherichia phage* JN02 | Rnl2 | +++ |
| 44 | YpRnl | *Yersinia phage* JC221 | Rnl2 | + |
| 45 | CpRnl | *Cronobacter phage* vB_CsaM_GAP161 | Rnl2 | ++ |
| 46 | AmeRnl | *Amsacta moorei entomopoxviru* | Rnl5 | ++ |
| 47 | CsRnl | *Chitinophaga sp.* S165 | Rnl5 | +++ |
| 48 | CAbRnl | *Candidatus Aminicenantes bacterium* | Rnl5 | +++ |
| 49 | MrRnl | *Massilia rubra* | Rnl5 | ++ |
| 50 | IbRnl | *Ignavibacteriaceae bacterium* | Rnl5 | +++ |
| 51 | TaRnl | *Thermoprotei archaeon* | Rnl3 | + |
| 52 | KpRnl | *Klebsiella pneumoniae* | Rnl1 | ++ |
| 53 | AbRnl | *Acidobacteria bacterium* | Rnl1 | +++ |
| 54 | SeRnl | *Salmonella enterica* | Rnl2 | +++ |
| 55 | YpRnl | *Yersinia phage* vB_YepM_ZN18 | Rnl2 | +++ |

| | | | | |
|---|---|---|---|---|
| Note: "+", "++", and "+++" in the table represented the catalytic activity of RNA ligases from low to high. | | | | |

### Embodiment 4

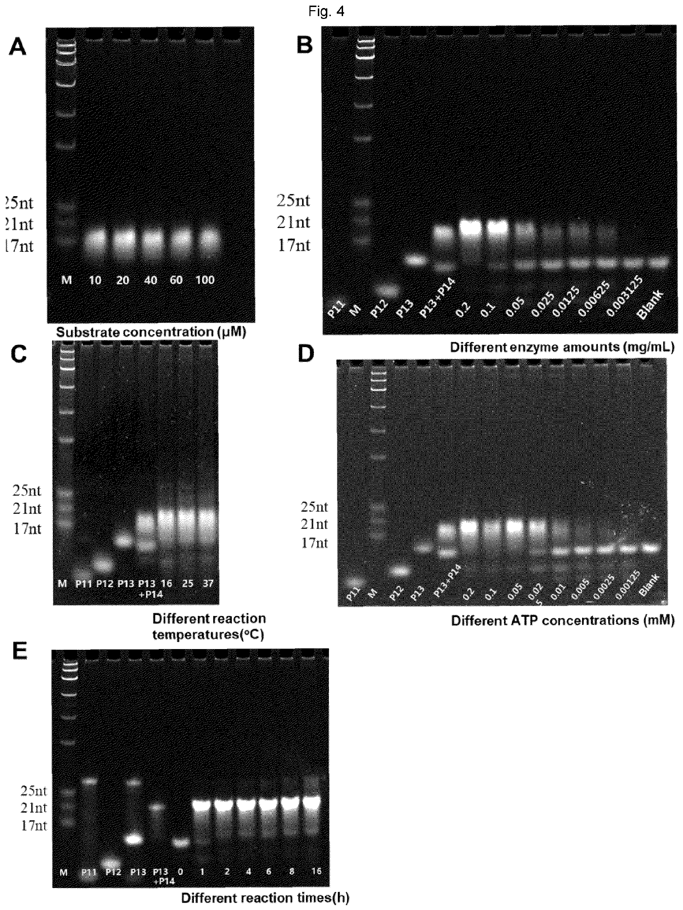

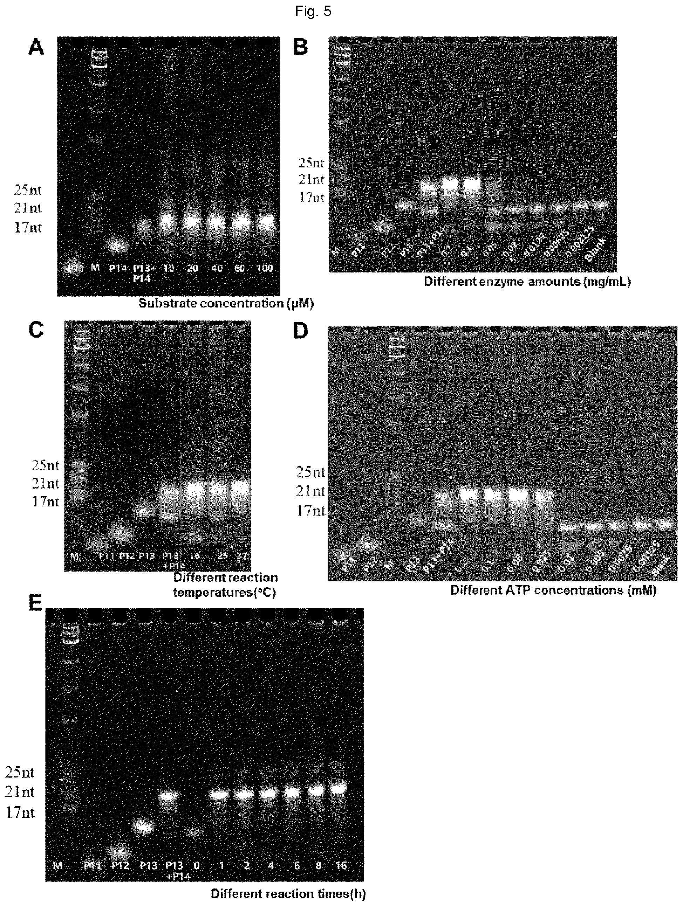

Enzyme linkage reactions of NgrRnl and JN02Rnl were optimized. The experiment was optimized with a single variable. The single variable included: substrate concentration, enzyme amount, reaction time, reaction temperature, ATP concentration and the like. As shown in Figs. 4 and 5. Herein, M represented ssRNA ladder, P11 represented a P11 substrate, P12 represented a P12 substrate, P13 represented a P13 standard substance, P13+P14 represented a double stranded nucleic acid formed by a combination of P13 and P14, and the blank was a reaction system without an enzyme added.

Fig. 4 showed a gel imaging diagram of an enzyme ligation reaction using NgrRnl, herein, in Fig. 4, A showed the effect of substrate concentration on the reaction, B showed the effect of enzyme amount on the reaction, C showed the effect of reaction temperature on the reaction, D showed the effect of ATP concentration on the reaction, and E showed the effect of reaction time on the reaction.

Fig. 5 showed a gel imaging diagram of an enzyme ligation reaction using JN02Rnl, herein, in Fig. 5, A showed the effect of substrate concentration on the reaction, B showed the effect of enzyme amount on the reaction, C showed the effect of reaction temperature on the reaction, D showed the effect of ATP concentration on the reaction, and E showed the effect of reaction time on the reaction.

The optimization results showed that when the substrate concentration was 400 µM, the maximum amount of the target product was generated and the conversion rate was relatively high. The optimization results of different enzyme amounts showed that the conversion rate was highest when the enzyme amount was 0.2 mg/mL. The experimental results of different reaction times showed that the reaction reached equilibrium at 2 h and the conversion rate was no longer increased. The optimization results of different temperatures showed that when the temperature was increased, the reaction might be promoted, but at the same time, more impurities were generated. Therefore, the optimal temperature for the reaction was still 16 °C. The optimization results of the ATP concentration in the reaction showed that at an enzyme amount of 0.2 mg/mL, 0.5 mM was sufficient.

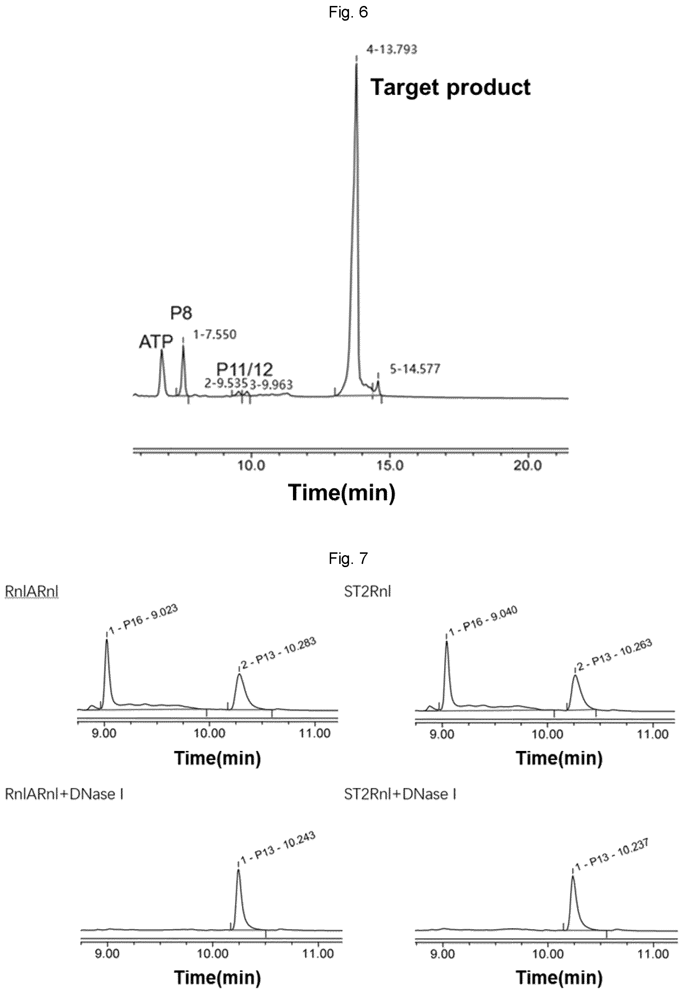

The optimized reaction conditions above, NgrRnl, 0.2 mg/mL of the enzyme amount, 400 µM of the substrate, 16 °C of the reaction temperature, and 0.5 mM of the ATP concentration, were used for the ligation reaction. After the reaction was ended, the target product was analyzed by UPLC. The UPLC analysis results were shown in Fig. 6. P9 and P10 were almost completely reacted, and the yield of the target product was about 90%. The purity of the target product after ion column purification might reach more than 90%. The main impurities were unreacted P8, as well as P11 formed by P8-P9 ligation and P12 formed by P9-P10.
P11 (9 nt): mA-fC-mG-fG-mG-fG-mU-fC-mA,
P12 (11 nt): mG-fG-mU-fC-mA-fC-mU-fG-mA-fG-mU (SEQ ID NO: 58).

### Embodiment 5

The present application attempted to use a DNA splint (single stranded DNA template) P16 (SEQ ID NO: 59) instead of an RNA splint P14 for a reaction.

P16: AACTCAGTGACCCCGTA (SEQ ID NO: 59).

The main purpose of using the DNA splint was that after the reaction was ended, the DNA splint in the system might be digested by DNasel, to obtain a high-purity single stranded RNA product. The UPLC analysis results showed that 18 proteins exhibited high RNA ligation activity. Herein, RnIB-BRnl, NgrRnl, SppRnl, CpMRnl, RnIARnl, KP15Rnl, KP27Rnl, WaRnl, and ST2Rnl not only generated the target product, but also generated very few impurities. The purity of the target product system might reach about 75%. After the reaction was ended, DNasel was added to the reaction system and incubated at 37 °C for 1 h. The reaction was stopped and it was sent for UPLC analysis after reaction treatment. The spectra of RnlARnl and ST2Rnl before and after splint digestion were shown in Fig. 7. The analysis results showed that DNasel might successfully digest the DNA splint.

### Embodiment 6

The present application also attempted a reaction of these enzymes ligating to a circular single RNA. The RNA substrate of the circular single RNA was a non-natural RNA strand with a length of 38 nt, and the sequence was as follows:

SEQ ID NO: 60: (H1, 38 nt, m represented 2'-OCH₃ modification)

mAmGmCmAmAmGmUmUmAmAmGmCmUmAmGmAmAmAmUmUmAmAmGmGmCmUmA
mTmGmUmUmAmAmUmUmAmGmA.

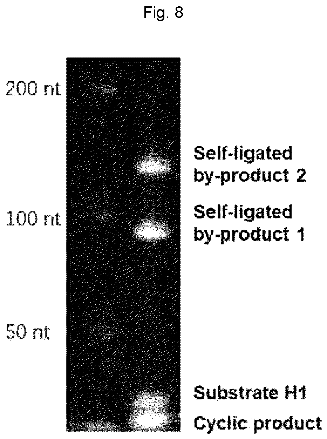

Reaction conditions: 20 µM of substrate H1 concentration, 0.2 mg/mL of the enzyme amount, ATP 1 mM, ligase reaction buffer, 16 °C of the reaction temperature, and 16 h of the reaction time. After the reaction was ended, mass spectrometry analysis was performed, and the analysis results showed that the target product was generated. RnlARnl ligase was taken as an example, the denaturing gel electrophoresis analysis results were shown in Fig. 8. RnlARnl had the highest activity to ligate the circular single RNA, which might reach more than 50%.

From the above description, it may be seen that the above embodiments of the present application achieve the following technical effects: the use of any one or more enzymes from the Rnl1, RnI2, Rnl3, and Rnl5 families, including but not limited to any one or more as shown in SEQ ID NO: 1~55, may achieve the ligation of the non-natural ribonucleotides. Compared to the prior art of using the solid-phase synthesis method to prepare the oligonucleotide, the efficiency is high and the cost is low. In addition, the above RNA ligases may simultaneously ligate the double stranded nucleic acid structures with a plurality of nicks, the reaction efficiency is high and the conversion rate is high. Most of the reactions are usually completed within 2 h, and the conversion rate is high.

The above are only preferred embodiments of the present application and are not intended to limit the present application. For those skilled in the art, the present application may have various modifications and variations. Any modifications, equivalent replacements, improvements and the like made within the spirit and principles of the present application shall be included within the scope of protection of the present application.

## Claims

1. A method for preparing an oligonucleotide with an RNA ligase, wherein the method comprises:
using the RNA ligase to ligate an RNA substrate to obtain the oligonucleotide;
the RNA ligase comprises one or more enzymes of the RNA ligase families Rnl1, RnI2, Rnl3, or Rnl5, and
the oligonucleotide comprises natural RNAs or non-natural RNAs.

2. The method according to claim 1, wherein the RNA ligase comprises any one or more of those having the amino acid sequence as shown in any one of SEQ ID NOs: 1 to 55;
preferably, the method comprises:
a) mixing, annealing and specifically binding a template strand to the RNA substrate to form a double stranded nucleic acid structure with nicks, and the number of the RNA substrate is 2 to 10, preferably 2 to 3;
b) ligating the nicks by a phosphodiester bond with the RNA ligase;
wherein the ribonucleotides ligated by the phosphodiester bond are all nonnatural ribonucleotides;
preferably, the nonnatural ribonucleotide comprises a ribonucleotide having one or more of a pentose ring 2' position modification, a phosphate α position modification or a base modification;
preferably, the pentose ring 2' position modification comprises 2'-methoxyl modification, 2'-fluoro modification, 2'-trifluoromethoxyl modification, 2'-methoxyethyl modification, 2'-allyl modification, 2'-amino modification or 2'-azido modification;
preferably, the phosphate α position modification comprises thio modification of phosphate at α position;
preferably, the base modification comprises the methylation modification and/or acetylation modification at any one or more of the N1, N5 or N6 positions of the base.

3. The method according to claim 2, wherein each of the RNA substrates is an RNA fragment of 2 to 100 nt, preferably 2 to 10 nt, and more preferably 4 to 6 nt;
preferably, the ribonucleotides are all the nonnatural ribonucleotides;
preferably, the template strand comprises a single stranded RNA template or a single stranded DNA template.

4. A method for preparing of a single stranded RNA, wherein the method comprises:
a) mixing, annealing and specifically binding a single stranded DNA template to RNA substrates to form a DNA-RNA hybrid double duplex with nicks, wherein the number of the RNA substrates is 2 to 10;
b) ligating the nicks by a phosphodiester bond with an RNA ligase to form a continuous DNA-RNA hybrid duplex;
c) removing the DNA strand in the continuous DNA-RNA hybrid duplex to obtain the single stranded RNA;
wherein, the RNA ligase comprises one or more enzymes of the RNA ligase families Rnl1, RnI2, Rnl3, or Rnl5; and
the ribonucleotides ligated by the phosphodiester bond are all non-natural ribonucleotides.

5. The method according to claim 4, wherein the RNA ligase comprises any one or more of those having the amino acid sequence as shown in any one of SEQ ID NOs: 1 to 55;
preferably, the DNA strand in the continuous DNA-RNA hybrid double duplex is removed by degradation with a DNase;
preferably, the DNase comprises DNasel, DNase1L1 or DNase1L2;
preferably, the RNA substrates is an RNA fragment of 2 to 100 nt, preferably 2 to 10 nt, and more preferably 4 to 6 nt in length;
preferably, the ribonucleotides are all the non-natural ribonucleotides;
preferably, the non-natural ribonucleotides comprises a ribonucleotide having one or more of a pentose ring 2' position modification , a phosphate α position modification or a base modification;
preferably, the pentose ring 2' position modification comprises 2'-methoxyl modification, 2'-fluoro modification, 2'-trifluoromethoxyl modification, 2'-methoxyethyl modification, 2'-allyl modification, 2'-amino modification or 2'-azido modification;
preferably, the phosphate α position modification comprises thio modification of phosphate at α position;
preferably, the base modification comprises the methylation modification and/or acetylation modification at any one or more of the N1, N5 or N6 positions of the base; and
preferably, the number of the RNA substrate is 2 to 3.

6. The method according to claim 4, wherein the single stranded RNA comprises a liner single stranded RNA, a semi-circular single stranded RNA or a circular single stranded RNA.

7. A method for preparing of a double stranded RNA, wherein the method comprises:
a) mixing, annealing and specifically binding a single stranded RNA template to RNA substrates to form a double stranded RNA with nicks, wherein the number of the RNA substrates is 2 to 10;
b) ligating the nicks by a phosphodiester bond with an RNA ligase to form the double stranded RNA;
wherein, the RNA ligase comprises one or more enzymes of the RNA ligase families Rnl1, RnI2, Rnl3, or Rnl5; and
the ribonucleotides ligated by the phosphodiester bond are all non-natural ribonucleotides.

8. The method according to claim 7, wherein the RNA ligase comprises any one or more of those having the amino acid sequence as shown in any one of SEQ ID NOs: 1 to 55;
preferably, the single stranded RNA template comprises a single stranded RNA prepared by the method of a single stranded RNA according to any one of claims 4 to 6; and
preferably, the double stranded RNA comprises a liner double stranded RNA, a semi-circular double stranded RNA or a circular double stranded RNA.

9. The method according to claim 7, wherein the RNA substrates are RNA fragments of 2 to 100 nt, preferably 2 to 10 nt, and more preferably 4 to 6 nt;
preferably, the number of the RNA substrates is 2 to 3.

10. The method according to claim 7, wherein the ribonucleotides of the RNA substrates are all the non-natural ribonucleotides;
preferably, the non-natural ribonucleotides comprises a ribonucleotide having one or more of a pentose ring 2' position modification, a phosphate α position modification or a base modification;
preferably, the pentose ring 2' position modification comprises 2'-methoxyl modification, 2'-fluoro modification, 2'-trifluoromethoxyl modification, 2'-methoxyethyl modification, 2'-allyl modification, 2'-amino modification or 2'-azido modification;
preferably, the phosphate α position modification comprises thio modification of phosphate at α position;
preferably, the base modification comprises the methylation modification and/or acetylation modification at any one or more of the N1, N5 or N6 positions of the base.
